(19) Europäisches Patentamt / European Patent Office / Office européen des brevets

(11) **EP 3 553 183 A1**

(12) **EUROPEAN PATENT APPLICATION**

(43) Date of publication:
16.10.2019 Bulletin 2019/42

(51) Int Cl.:
*C12Q 1/6832* (2018.01)

(21) Application number: **19168606.2**

(22) Date of filing: **11.04.2019**

| | |
|---|---|
| (84) Designated Contracting States:<br>**AL AT BE BG CH CY CZ DE DK EE ES FI FR GB GR HR HU IE IS IT LI LT LU LV MC MK MT NL NO PL PT RO RS SE SI SK SM TR**<br>Designated Extension States:<br>**BA ME**<br>Designated Validation States:<br>**KH MA MD TN**<br><br>(30) Priority: **12.04.2018 US 201815952045**<br><br>(71) Applicant: **Cellmax, Ltd.**<br>**Grand Cayman KY-1-1 111 (KY)** | (72) Inventors:<br>• **Mei, Rui**<br>**Santa Clara, CA 95051 (US)**<br>• **Lucas, Julian**<br>**San Jose, CA 95125 (US)**<br>• **Gupta, Pratyush**<br>**Mountain View, CA 94040 (US)**<br><br>(74) Representative: **IP Sextant s.r.l.**<br>**Via A. Salandra, n.18**<br>**00187 Rome (IT)** |

(54) **HYBRIDIZATION SOLUTION FOR CAPTURING A NUCLEIC ACID INCLUDING A TARGET OLIGONUCLEOTIDE SEQUENCE**

(57)    Provided herein are methods of capturing a nucleic acid comprising a target oligonucleotide sequence from a library of nucleic acid that include: contacting a library of nucleic acids comprising a nucleic acid comprising a target oligonucleotide sequence with a probe comprising a sequence that is complementary to the target oligonucleotide sequence, wherein the contacting is performed in a tetramethylammonium chloride (TMAC)-based buffer at a temperature of about 60 °C to about 70 °C, and the contacting results in the hybridization of the target oligonucleotide sequence to the sequence that is complementary to the target oligonucleotide sequence, to thereby generate a hybridization product; and isolating the hybridization product from nucleic acids in the library that do not comprise the target oligonucleotide sequence. Also provided are compositions useful for performing these methods.

FIG. 1

**Description**

**TECHNICAL FIELD**

The present disclosure relates to the fields of oligonucleotide hybridization and the purification of nucleic acids.

**BACKGROUND**

Next-generation sequencing allows countless genomes to be sequenced in a fraction of the time that it once took. Despite these technical advances, whole genome sequencing remains very expensive and as a result target enrichment is necessary.

There are two types of target enrichment strategies: Amplicon based and Hybridization based. Amplicon strategy relies on enrichment via Polymerase Chain Reaction (PCR) based amplification of target using short complementary nucleotide sequences called primers. However, they result in missing fragments of DNA thus missing variants and introducing errors. The hybridization strategy on the other hand relies on binding fragments of DNA based on complementarity resulting in efficient capture of all variants for a given target. However, the hybridization strategy suffers from problems such as strand bias, uneven coverage and inefficient binding and capture. Without wishing to be bound by theory, the present inventors developed a buffer and hybridization/capture method to overcome these challenges.

**SUMMARY**

The present invention is based on the discovery that the use of new tetramethylammonium chloride (TMAC)-based buffer in the methods provided herein provide for a low off-target rate and a more even target coverage than other methods known in the art.

In view of this discovery, provided herein are methods of capturing a nucleic acid including a target oligonucleotide sequence that include contacting a library of nucleic acids including a nucleic acid including a target oligonucleotide sequence with a probe including a sequence that is complementary to the target oligonucleotide sequence, where the contacting is performed in a TMAC-based buffer at a temperature of about 60 °C to about 70 °C, and the contacting results in the hybridization of the target oligonucleotide sequence to the sequence that is complementary to the target oligonucleotide sequence, to thereby generate a hybridization product; and isolating the hybridization product from nucleic acids in the library that do not comprise the target oligonucleotide sequence.

Provided herein are methods of capturing a nucleic acid comprising a target oligonucleotide sequence from a library of nucleic acids, that include: contacting a library of nucleic acids comprising a nucleic acid comprising a target oligonucleotide sequence with a probe comprising a sequence that is complementary to the target oligonucleotide sequence, wherein the contacting is performed in a tetramethylammonium chloride (TMAC)-based buffer at a temperature of about 60 °C to about 70 °C, and the contacting results in the hybridization of the target oligonucleotide sequence to the sequence that is complementary to the target oligonucleotide sequence, to thereby generate a hybridization product; and isolating the hybridization product from nucleic acids in the library that do not comprise the target oligonucleotide sequence.

In some embodiments, the contacting step is performed at a temperature of about 64 °C to about 66 °C.

In some embodiments, the hybridization product is a RNA-DNA product.

In some embodiments, the TMAC-based buffer comprises about 0.5 M to about 4.0 M TMAC.

In some embodiments of any of the methods described herein, the TMAC-based buffer further includes one or more of: about 10 mM to about 200 mM 2-amino-2-(hydroxymethyl)propane-1,3-diol (Tris); about 1X to about 5x Denhardt's Solution; about 0.01% to about 0.2% Tween-20; about 0.5 mM to about 10 mM ethylenedioaminetetraacetic acid (EDTA); and about 0.5% to about 25% (v/v) formamide.

In some embodiments of any of the methods described herein, the TMAC-based buffer further includes: about 10 mM to about 200 mM 2-amino-2-(hydroxymethyl)propane-1,3-diol (Tris); about 1X to about 5x Denhardt's Solution; about 0.01% to about 0.2% Tween-20; about 0.5 mM to about 10 mM ethylenedioaminetetraacetic acid (EDTA); and about 0.5% to about 25% (v/v) formamide.

In some embodiments, the TMAC-based buffer includes: about 40 mM to about 60 mM 2-amino-2-(hydroxymethyl)propane-1,3-diol (Tris); about 2X to about 3X Denhardt's Solution; about 0.01% to about 0.05% Tween-20; about 0.5 mM to about 7 mM ethylenedioaminetetraacetic acid (EDTA); and about 0.5% to about 25% (v/v) formamide.

In some embodiments of any of the methods described herein, the TMAC-based buffer includes about 2.7 M TMAC, about 50 mM Tris (pH 8.0), about 2.5X Denhardt's Solution, about 0.010% Tween-20, about 6 mM EDTA, and about 20% formamide.

In some embodiments of any of the methods described herein, the TMAC-based buffer includes about 5.4 M TMAC, about 100 mM Tris (pH 8.0), about 5X Denhardt's Solution, about 0.02% Tween-20, and about 12 mM EDTA.

In some embodiments of any of the methods described herein, the contacting step is performed for about 1 hour to about 48 hours. In some embodiments, the contacting step is performed for about 10 hours to about 20 hours.

In some embodiments of any of the methods described herein, the probe comprises a tag that is positioned internally or at the 5' or 3' end of the nucleic acid sequence of the probe. In some embodiments, the tag is biotin, or a variant thereof.

In some embodiments of any of the methods described herein, the isolating is performed using a bead. In some embodiments, the isolating is performed using a bead comprising a moiety that specifically binds to the tag.

In some embodiments of any of the methods described herein, the method further includes at least one washing step after the contacting step and the isolating step. In some embodiments, the at least one washing step comprises the use of a low stringency buffer and a high stringency buffer.

In some embodiments, the at least one washing step comprises washing using a low stringency buffer, at a temperature of about 16 °C to about 30 °C, for about 1 minute to about 10 hours.

In some embodiments of any of the methods described herein, the washing using a low stringency buffer is performed at a temperature of about 23 °C to about 27 °C, for about 5 minutes to about 40 minutes.

In some embodiments, the low stringency buffer includes a buffered solution and optionally, a detergent. In some embodiments, the low stringency buffer includes saline-sodium citrate (SSC) buffer and optionally, sodium dodecyl sulfate (SDS). In some embodiments, the low stringency buffer includes about 0.5X to about 2.5X SSC, and 0% to about 0.15% SDS.

In some embodiments of any of the methods described herein, the at least one washing step comprises washing using a high stringency buffer, at a temperature of about 45 °C to about 75 °C, for about 1 minute to about 10 hours. In some embodiments, the washing using a high stringency buffer is performed at a temperature of about 45 °C to about 75 °C, for about 1 minute to about 4 hours.

In some embodiments, the high stringency buffer comprises about 0.1X to about 0.5X SSC, and optionally, a detergent. In some embodiments, the high stringency buffer comprises about 0.15X to about 0.35X SSC, and optionally, a detergent. In some embodiments, the high stringency buffer comprises about 0% to about 0.15% SDS.

Also provided herein are compositions that include a liquid, where the liquid includes about 0.5 M to about 8.0 M TMAC, about 10 mM to about 200 mM Tris (pH 8.0), about 1X to about 5X Denhardt's solution, about 0.01% to about 0.2% Tween-20, about 0.5 mM to about 15 mM EDTA and about 0.5% to about 25% formamide (v/v).

In some embodiments of any of the compositions described herein, the liquid includes about 2.0 M to about 6.0 M TMAC. In some embodiments of any of the compositions described herein, the liquid includes about 5.0 M to about 6.0 M TMAC. In some embodiments of any of the compositions described herein, the liquid includes about 5.4 M TMAC.

In some embodiments of any of the compositions described herein, the liquid includes about 40 mM to about 60 mM Tris (pH 8.0). In some embodiments of any of the compositions described herein, the liquid includes about 100 mM Tris (pH 8.0).

In some embodiments of any of the components described herein, the liquid includes about 2X to about 3X Denhardt's Solution. In some embodiments of any of the compositions described herein, the liquid includes about 5X Denhardt's Solution.

In some embodiments of any of the compositions described herein, the liquid includes about 0.01% to about 0.05% Tween-20. In some embodiments of any of the compositions described herein, the liquid includes about 5 mM to about 15 mM EDTA.

In some embodiments of any of the compositions described herein, the liquid includes about 10% to about 25% formamide (v/v). In some embodiments of any of the compositions described herein, the liquid includes about 20% formamide (v/v).

In some embodiments of any of the compositions described herein, the liquid includes about 2.7 M TMAC, about 50 mM Tris (pH 8.0), about 2.5X Denhardt's Solution, about 0.010% Tween-20, about 6 mM EDTA, and about 20% formamide. In some embodiments of any of the compositions described herein, the composition consists of the liquid.

Also provided herein are kits that include any one of the compositions described herein. Some embodiments of any of the kits described herein further include instructions to perform any of the methods described herein.

As used herein the term "off-target" refers to the enrichment of a nucleic acid that was not intended to be enriched (e.g., a nucleic acid that does not include the target sequence). Such "off-target" binding may lower sensitivity due to missed regions, increase false positive due to lower coverage causing poor confidence, and/or cost more money. In some embodiments, off-target binding can have deleterious effects.

As used herein the term "target coverage" refers to the sequencing coverage of a target region (e.g., a gene locus or a portion of a gene locus of interest).

As used herein the term "sequencing coverage" or "sequencing depth" refers to the number of sequenced products which cover a targeted nucleotide sequence in a library.

As used herein the term "GC content" refers to the content of guanine nucleotides (Gs) and cytosine nucleotides (Cs) in a DNA oligonucleotide over a specified region. The GC content is usually expressed as a percentage. Regions with many Gs and Cs, as opposed to As and Ts, are said to have a high GC content. High GC content is a GC percentage of about 60% and above, e.g., about 62% and above, about 64% and above, about 66% and above, about 68% and above, about 70% and above, about 72% and above, about 74% and above, about 76% and above, about 78% and above, about 80% and above, about 82% and above, about 84% and above, about 86% and above, about 88% and above, about 90% and above, about 92% and above, about 94% and above, about 96% and above, about 98% and above, or about 60%, about 61%, about 62%, about 63%, about 64%, about 65%, about 66%, about 67%, about 68%, about 69%, about 70%, about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, or 100%. Low GC content is a GC percentage of about 59% and below, about 58% and below, about 56% and below, about 54% and below, about 52% and below, about 50% and below, about 48% and below, about 46% and below, about 44% and below, about 42% and below, about 40% and below, about 38% and below, about 36% and below, about 34% and below, about 32% and below, about 30% and below, about 28% and below, about 26% and below, about 24% and below, about 22% and below, about 20% and below, about 18% and below, about 16% and below, about 14% and below, about 12% and below, about 10% and below, about 8% and below, about 6% and below, about 4% and below, about 2% and below, about 1% and below, or about 0.5%, about 1%, about 2%, about 3%, about 4%, about 5%, about 6%, about 7%, about 8%, about 9%, about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%, about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, or about 59%.

The term "primer" refers to an oligonucleotide that is capable of acting as a point of initiation for the 5' to 3' synthesis of an extension product that is complementary to one or more nucleic acids (e.g., a target nucleic acid strand, e.g., a target oligonucleotide sequence). The extension product(s) is/are synthesized in the presence of appropriate nucleotides and an agent for polymerization, such as a DNA polymerase, in an appropriate buffer and at a suitable temperature.

Unless otherwise defined, all technical and scientific terms used herein have the same meaning as commonly understood by one of ordinary skill in the art to which this invention belongs. Methods and materials are described herein for use in the present invention; other, suitable methods and materials known in the art can also be used. The materials, methods, and examples are illustrative only and not intended to be limiting.

Other features and advantages of the invention will be apparent from the following detailed description and figures, and from the claims.

## BRIEF DESCRIPTION OF DRAWINGS

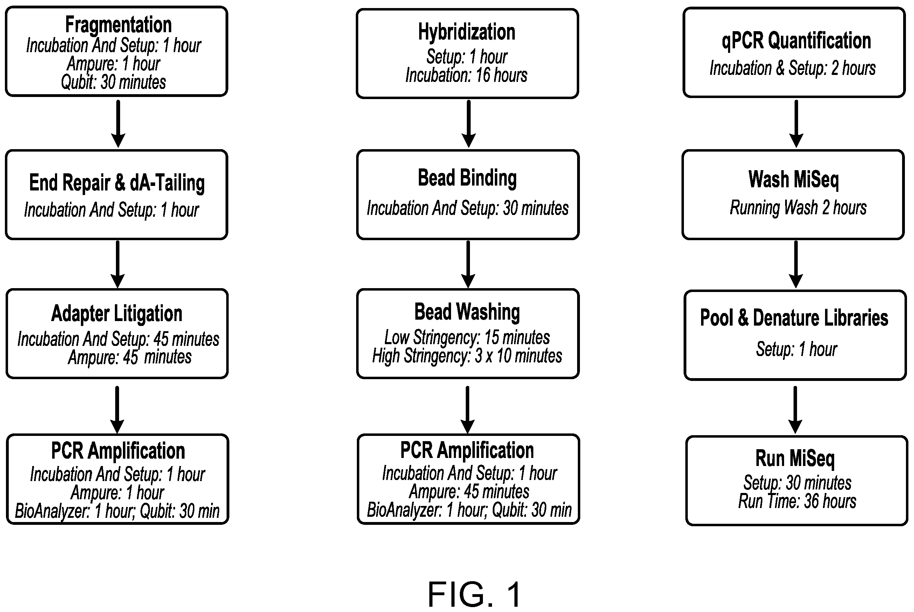

FIG. 1 is a schematic of an exemplary process: the DNA fragmentation, end repair and dA-tailing, adapter ligation, and PCR amplification steps are shown on the left; the capturing steps and PCR amplification step are shown in the middle; and the sequencing preparation and execution steps are shown on the right.

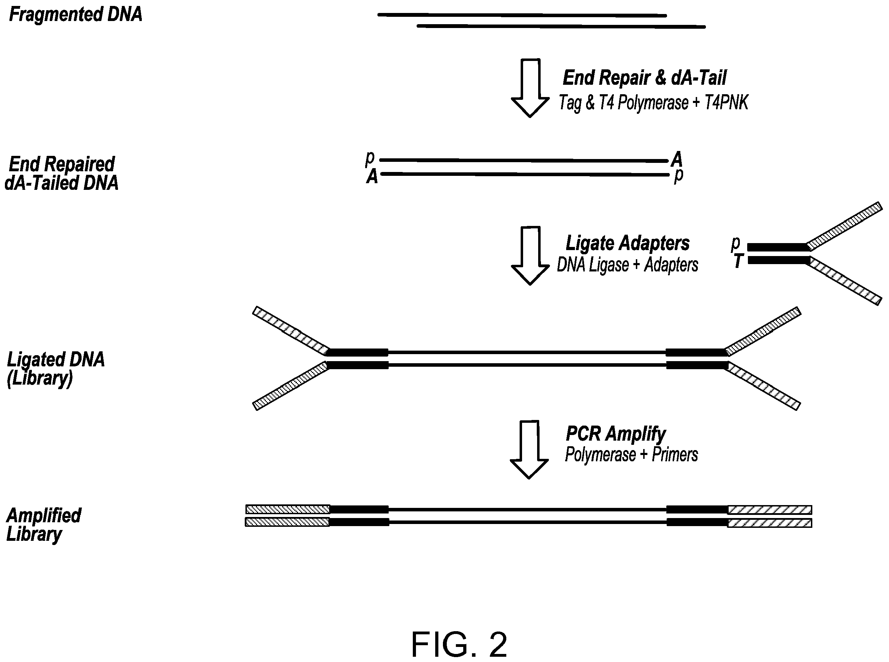

FIG. 2 is a representative schematic representation of the DNA fragmentation, end repair and dA-tailing, adapter ligation, and PCR amplification steps that converts fragmented genomic DNA into PCR-amplified libraries with Illumina adapters. Briefly, fragmented DNA is blunt-ended, dA-tailed, and 5' phosphorylated by a cocktail of enzymes. The DNA is then ligated to branched adapters. The adapter-ligated libraries are amplified, which increases the amount of DNA available, and linearizes the adaptors.

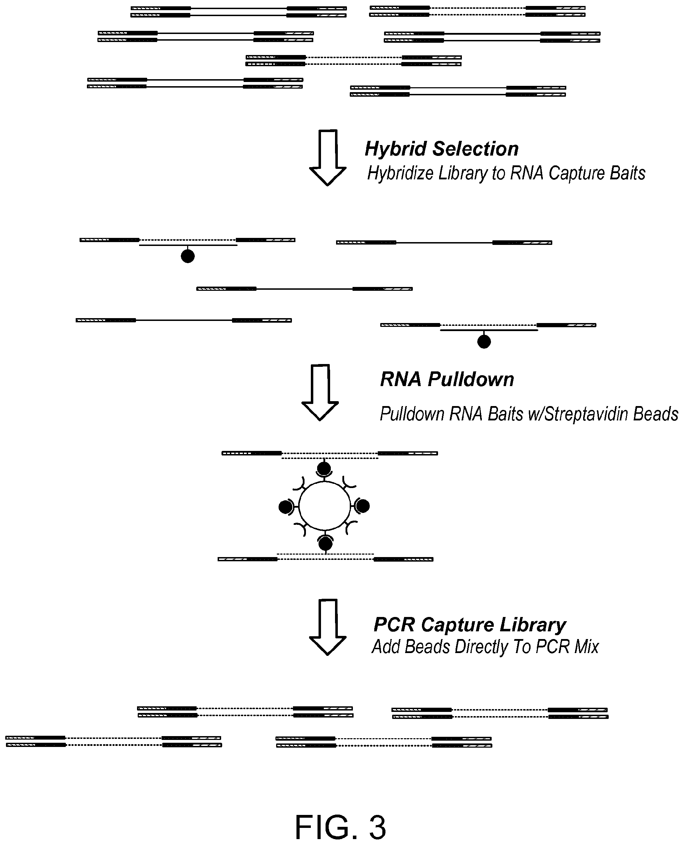

FIG. 3 is a schematic showing the hybridization of PCR-amplified libraries with Illumina adapters that are hybridized against RNA probes that are covalently linked to biotin (RNA Capture Baits). The RNA-DNA hybridization products are pulled down with streptavidin beads that bind to the biotin tag in the probes. Beads with captured library sequences are washed to remove library molecules that are not targeted, and the enriched libraries are amplified by polymerase chain reaction (PCR).

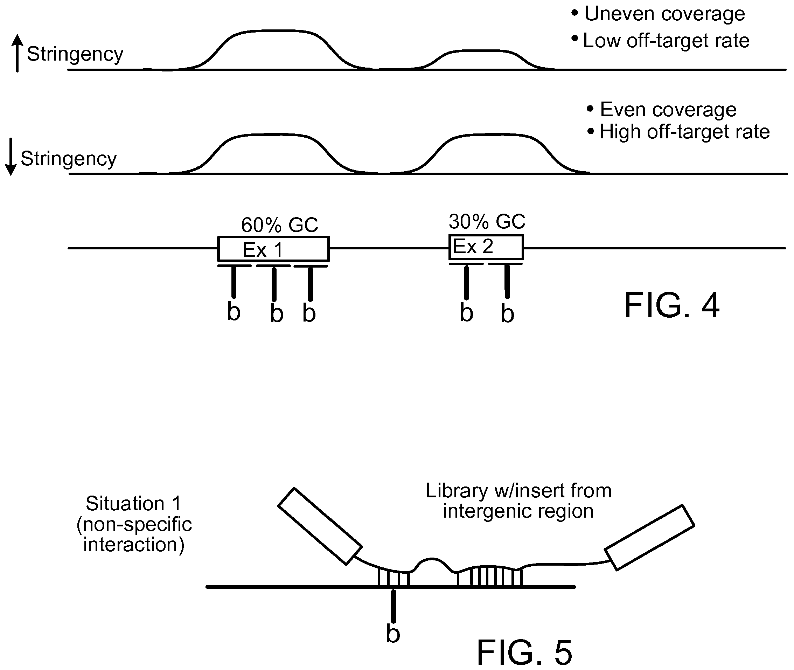

FIG. 4 is a schematic representation of a target sequence of high GC content (60% GC) and low GC content (30% GC) in relation to hybridization stringency, coverage, and off-target rate.

FIG. 5 is a schematic of a probe comprising a sequence that is complementary to a target oligonucleotide sequence that depicts a non-specific interaction. The probe includes a tag that is indicated by the symbol "b".

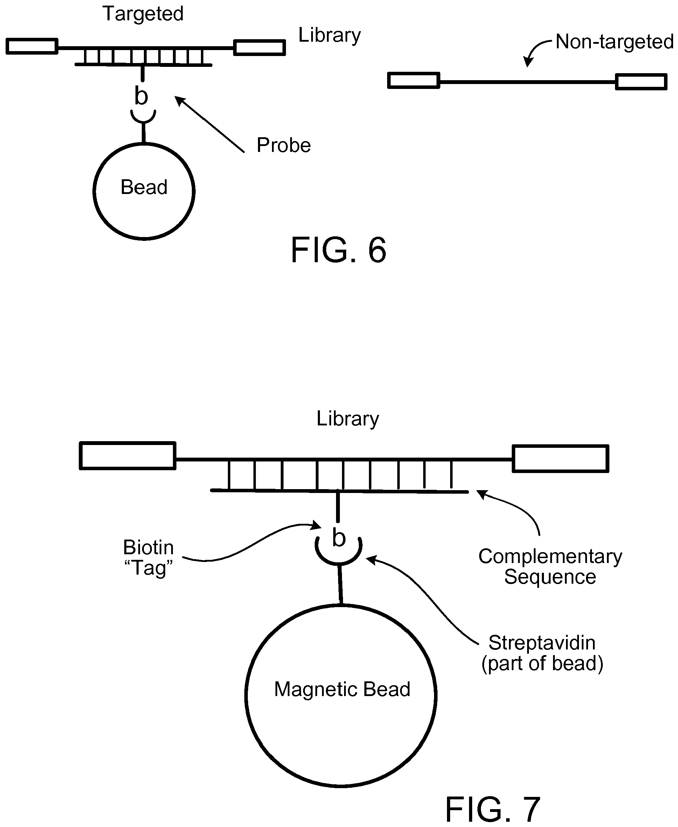

FIG. 6 is an exemplary drawing of a hybridization product in which a target oligonucleotide sequence has hybridized to a probe that includes a sequence that is complementary to the target oligonucleotide sequence and has an internal tag. The internal tag of the probe is bound by a bead that specifically binds to the internal tag. A nucleic acid sequence that does not include the target oligonucleotide sequence is shown to the right of the hybridization product.

FIG. 7 is an exemplary drawing of a hybridization product in which a target oligonucleotide sequence has hybridized to a probe that includes a sequence that is complementary to the target oligonucleotide sequence and has an internal biotin tag. The internal biotin tag of the probe is bound by a magnetic bead that is covalently conjugated to streptavidin, and the streptavidin specifically binds to the biotin tag.

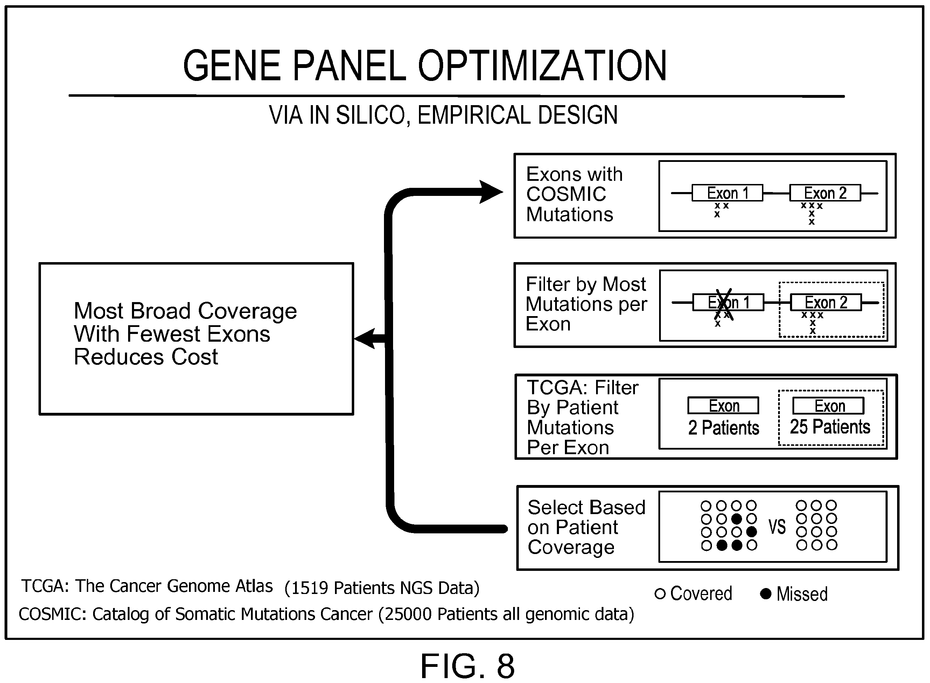

FIG. 8 is a schematic diagram describing the probe selection and formulation strategy that involves selecting exons with most number of variants covering maximum number of patients based on the TGCA and COSMIC databases.

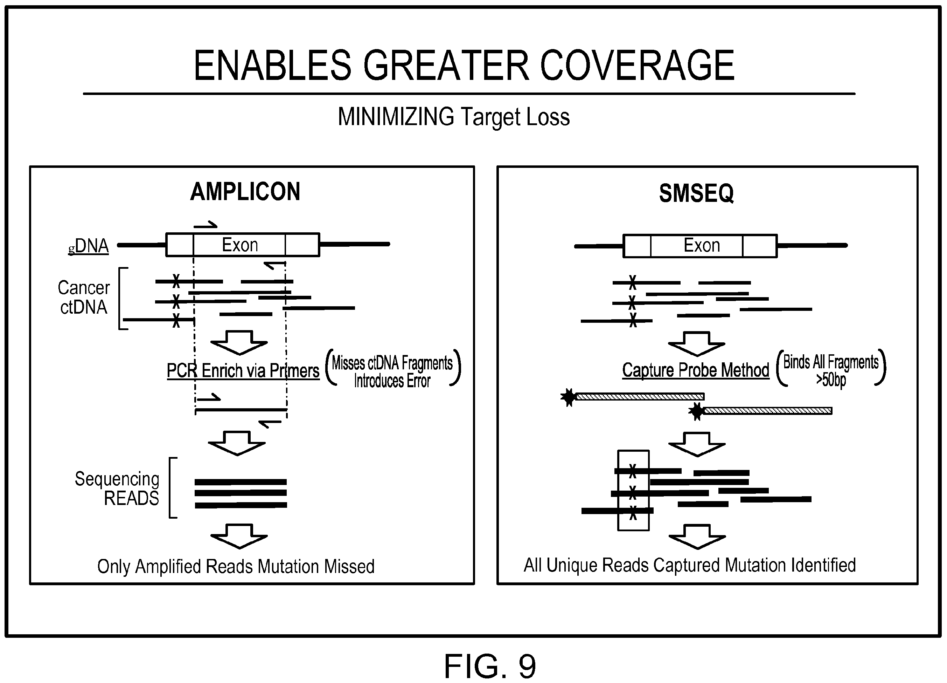

FIG. 9 is an exemplary drawing explaining differences between Amplicon and Hybridization based target enrichment strategies and advantages of the latter.

## DETAILED DESCRIPTION

The present inventors developed a novel tetramethylammonium chloride (TMAC) based hybridization buffer and combined it with enhanced methodology described below. TMAC is a quaternary salt that helps to stabilize the Adenine (A) and Thiamine (T) nucleotide regions thereby improving hybridization efficiency considerably at 60-70'C.

Provided herein are methods of capturing a nucleic acid including a target oligonucleotide sequence from a library of nucleic acids that include: contacting a library of nucleic acids including a nucleic acid that includes a target oligonucleotide sequence with a probe that includes a sequence that is complementary to the target oligonucleotide sequence, wherein the contacting is performed in a tetramethylammonium chloride (TMAC)-based buffer at a temperature of about 60 °C to about 70 °C, and the contacting results in the hybridization of the target oligonucleotide sequence to the sequence that is complementary to the target oligonucleotide sequence, to thereby generate a hybridization product; and isolating the hybridization product from the library.

Also provided herein are any of the TMAC-based buffers described herein. Such TMAC-based buffers can be used in any of the methods described herein.

The methods provide for an analytical sensitivity (as described herein) of greater than 95%, greater than 95.5%, greater than 96.0%, greater than 96.5%, greater than 97.0%, greater than 97.5%, greater than 98.0%, greater than 98.5%, greater than 98.6%, greater than 98.7%, greater than 98.8%, greater than 98.9%, greater than 99.0%, greater than 99.1%, greater than 99.2%, greater than 99.3%, greater than 99.4%, greater than 99.5%, greater than 99.6%, greater than 99.7%, greater than 99.8%, greater than 99.9%, or 100%.

The methods provide for a reproducibility (as described herein) of greater than 95%, greater than 95.5%, greater than 96.0%, greater than 96.5%, greater than 97.0%, greater than 97.5%, greater than 98.0%, greater than 98.5%, greater than 98.6%, greater than 98.7%, greater than 98.8%, greater than 98.9%, greater than 99.0%, greater than 99.1%, greater than 99.2%, greater than 99.3%, greater than 99.4%, greater than 99.5%, greater than 99.6%, greater than 99.7%, greater than 99.8%, greater than 99.9%, or 100%.

The methods provide for an average sequencing cover of greater than 95%, greater than 95.5%, greater than 96.0%, greater than 96.5%, greater than 97.0%, greater than 97.5%, greater than 98.0%, greater than 98.5%, greater than 98.6%, greater than 98.7%, greater than 98.8%, greater than 98.9%, greater than 99.0%, greater than 99.1%, greater than 99.2%, greater than 99.3%, greater than 99.4%, greater than 99.5%, greater than 99.6%, greater than 99.7%, greater than 99.8%, greater than 99.9%, or 100%.

Non-limiting aspects of these methods are described below, and can be used in any combination without limitation. Additional aspects of these methods are known in the art.

## Libraries

The methods provided herein allow for the capture of a nucleic acid including a target oligonucleotide sequence from a library of nucleic acids that include a nucleic acid including a target oligonucleotide sequence. For example, a library can include a plurality of different nucleic acids, where each nucleic acid includes a different nucleic acid sequence. In some embodiments, each nucleic acid within a library has the same length or has approximately the same length. In some embodiments, each nucleic acid within a library is of a different length. In some embodiments, at least two nucleic acids within a library have a different length.

A library of nucleic acids can be a library of double-stranded DNAs, a library of single stranded DNAs, a library of single-stranded RNAs, a library of double-stranded RNAs, or a library of double-stranded nucleic acids made of one strand of DNA and one strand of RNA.

In some embodiments, the nucleic acid(s) in a library can be of chromosomal, plasmid, genomic, mitochondrial, exosomal, cell-free DNA, cellular (e.g., mammalian cellular), or viral origin. In some embodiments, one or both strands of a double-stranded nucleic acid molecule (e.g., any of the double-stranded nucleic acids described herein) can be captured using any of the methods described herein.

A library of nucleic acids may include a plurality of double-stranded nucleic acids (e.g., double-stranded DNAs, double-stranded RNAs, or double-stranded nucleic acids made of one strand of DNA and one strand of RNA) having a total length of, e.g., about 20 base pairs (bp) to about 5,000 bp, about 20 bp to about 4,000 bp, about 20 bp to about 3,000 bp, about 20 bp to about 2,000 bp, about 20 bp to about 1,500 bp, about 20 bp to about 1,000 bp, about 20 bp to about 500 bp, about 20 bp to about 100 bp, about 20 bp to about 60 bp, about 20 bp to about 40 bp, about 100 bp to about 5,000 bp, about 100 bp to about 4,000 bp, about 100 bp to about 2,000 bp, about 100 bp to about 1,000 bp, about 100 bp to about 500 bp, about 100 bp to about 250 bp, about 100 bp to about 200 bp, about 250 bp to about 5,000 bp, about 250 bp to about 1,000 bp, about 250 bp to about 500 bp, about 500 bp to about 5,000 bp, about 500 bp to about 2,000 bp, about 500 bp to about 1,000 bp, about 1,000 bp to about 5,000 bp, about 1,000 bp to about 2,000 bp, about 1,500 bp to about 5,000 bp, about 1,500 bp to about 2,000 bp, about 2,000 bp to about 5,000 bp, about 2,000 bp to about 4,000 bp, about 3,000 bp to about 5,000 bp, about 3,000 bp to about 4,000 bp, or about 4,500 bp to about 5,000 bp.

A library of nucleic acids may include a plurality of single-stranded nucleic acids (e.g., single-stranded DNAs or single-stranded RNAs) having a total length of, e.g., about 20 nucleotides (nt) to about 5,000 nt, about 20 bp to about 4,000 bp, about 20 bp to about 3,000 bp, about 20 bp to about 2,000 bp, about 20 bp to about 1,500 bp, about 20 bp to about 1,000 bp, about 20 bp to about 500 bp, about 20 bp to about 100 bp, about 20 bp to about 60 bp, about 20 bp to about 40 bp, about 100 bp to about 5,000 bp, about 100 bp to about 4,000 bp, about 100 bp to about 2,000 bp, about 100 bp to about 1,000 bp, about 100 bp to about 500 bp, about 100 bp to about 250 bp, about 100 bp to about 200 bp, about 250 bp to about 5,000 bp, about 250 bp to about 1,000 bp, about 250 bp to about 500 bp, about 500 bp to about 5,000 bp, about 500 bp to about 2,000 bp, about 500 bp to about 1,000 bp, about 1,000 bp to about 5,000 bp, about 1,000 bp to about 2,000 bp, about 1,500 bp to about 5,000 bp, about 1,500 bp to about 2,000 bp, about 2,000 bp to about 5,000 bp, about 2,000 bp to about 4,000 bp, about 3,000 bp to about 5,000 bp, about 3,000 bp to about 4,000 bp, or about 4,500 bp to about 5,000 bp.

A library of nucleic acids may include a plurality of at least $1 \times 10^3$ different nucleic acids, at least $1 \times 10^4$ different nucleic acids, at least $1 \times 10^5$ different nucleic acids, at least $1 \times 10^6$ different nucleic acids, at least $1 \times 10^7$ different nucleic acids, at least $1 \times 10^8$ different nucleic acids, at least $1 \times 10^9$ different nucleic acids, at least $1 \times 10^{10}$ different nucleic acids, at least $1 \times 10^{11}$ different nucleic acids, at least $1 \times 10^{12}$ different nucleic acids, at least $1 \times 10^{13}$ different nucleic acids, at least $1 \times 10^{14}$ different nucleic acids, or at least $1 \times 10^{15}$ different nucleic acids. For example, any of the libraries described herein can include a plurality of, e.g., about $1.0 \times 10^2$ different nucleic acids to about $1.0 \times 10^9$ different nucleic acids, about $1.0 \times 10^2$ different nucleic acids to about $0.5 \times 10^9$ different nucleic acids, about $1.0 \times 10^2$ different nucleic acids to about $1.0 \times 10^8$ different nucleic acids, about $1.0 \times 10^2$ different nucleic acids to about $0.5 \times 10^8$ different nucleic acids, about $1.0 \times 10^2$ different nucleic acids to about $1.0 \times 10^7$ different nucleic acids, about $1.0 \times 10^2$ different nucleic acids to about $0.5 \times 10^7$ different nucleic acids, about $1.0 \times 10^2$ different nucleic acids to about $1.0 \times 10^6$ different nucleic acids, about $1.0 \times 10^2$ different nucleic acids to about $0.5 \times 10^6$ different nucleic acids, about $1.0 \times 10^2$ different nucleic acids to about $1.0 \times 10^5$ different nucleic acids, about $1.0 \times 10^2$ different nucleic acids to about $0.5 \times 10^5$ different nucleic acids, about $1.0 \times 10^2$ different nucleic acids to about $1.0 \times 10^4$ different nucleic acids, about $1.0 \times 10^2$ different nucleic acids to about $0.5 \times 10^4$ different nucleic acids, about $1.0 \times 10^3$ different nucleic acids to about $0.5 \times 10^9$ different nucleic acids, about $1.0 \times 10^3$ different nucleic acids to about $1.0 \times 10^8$ different nucleic acids, about $1.0 \times 10^3$ different nucleic acids to about $0.5 \times 10^8$ different nucleic acids, about $1.0 \times 10^3$ different nucleic acids to about $1.0 \times 10^7$ different nucleic acids, about $1.0 \times 10^3$ different nucleic acids to about $0.5 \times 10^7$ different nucleic acids, about $1.0 \times 10^3$ different nucleic acids to about $1.0 \times 10^6$ different nucleic acids, about $1.0 \times 10^3$ different nucleic acids to about $0.5 \times 10^6$ different nucleic acids, about $1.0 \times 10^3$ different nucleic acids to about $1.0 \times 10^5$ different nucleic acids, about $1.0 \times 10^3$ different nucleic acids to about $0.5 \times 10^5$ different nucleic acids, about $1.0 \times 10^3$ different nucleic acids to about $1.0 \times 10^4$ different nucleic acids, about $1.0 \times 10^3$ different nucleic acids to about $0.5 \times 10^4$ different nucleic acids, about $0.5 \times 10^4$ different nucleic acids to about $1.0 \times 10^9$ different nucleic acids, about $0.5 \times 10^4$ different nucleic acids to about $0.5 \times 10^9$ different nucleic acids, about $0.5 \times 10^4$ different nucleic acids to about $1.0 \times 10^8$ different nucleic acids, about $0.5 \times 10^4$ different nucleic acids to about $0.5 \times 10^8$ different nucleic acids, about $0.5 \times 10^4$ different nucleic acids to about $1.0 \times 10^7$ different nucleic acids, about $0.5 \times 10^4$ different nucleic acids to about $0.5 \times 10^7$ different nucleic acids, about $0.5 \times 10^4$ different nucleic acids to about $1.0 \times 10^6$ different nucleic acids, about $0.5 \times 10^4$ different nucleic acids to about $0.5 \times 10^6$ different nucleic acids, about $0.5 \times 10^4$ different nucleic acids to about $1.0 \times 10^5$ different nucleic acids, about $0.5 \times 10^4$ different nucleic acids to about $0.5 \times 10^5$ different nucleic acids, about $0.5 \times 10^4$ different nucleic acids to about $1.0 \times 10^4$ different nucleic acids, about $1.0 \times 10^4$ different nucleic acids to about $1.0 \times 10^9$ different nucleic acids, about $1.0 \times 10^4$ different nucleic acids to about $0.5 \times 10^9$ different nucleic acids, about $1.0 \times 10^4$ different nucleic acids to about $1.0 \times 10^8$ different nucleic acids, about $1.0 \times 10^4$ different nucleic acids to about $0.5 \times 10^8$ different nucleic acids, about $1.0 \times 10^4$ different nucleic acids to about $1.0 \times 10^7$ different nucleic acids, about $1.0 \times 10^4$ different nucleic acids to about $0.5 \times 10^7$ different nucleic acids, about $1.0 \times 10^4$ different nucleic acids to about $1.0 \times 10^6$ different nucleic acids, about $1.0 \times 10^4$ different nucleic acids to about $0.5 \times 10^6$ different nucleic acids, about $1.0 \times 10^4$ different nucleic acids to about $1.0 \times 10^5$ different nucleic acids, about $1.0 \times 10^4$ different nucleic acids to about $0.5 \times 10^5$ different nucleic acids, about

0.5 x $10^5$ different nucleic acids to about 1.0 x $10^9$ different nucleic acids, about 0.5 x $10^5$ different nucleic acids to about 0.5 x $10^9$ different nucleic acids, about 0.5 x $10^5$ different nucleic acids to about 1.0 x $10^8$ different nucleic acids, about 0.5 x $10^5$ different nucleic acids to about 0.5 x $10^8$ different nucleic acids, about 0.5 x $10^5$ different nucleic acids to about 1.0 x $10^7$ different nucleic acids, about 0.5 x $10^5$ different nucleic acids to about 0.5 x $10^7$ different nucleic acids, about 0.5 x $10^5$ different nucleic acids to about 1.0 x $10^6$ different nucleic acids, about 0.5 x $10^5$ different nucleic acids to about 0.5 x $10^6$ different nucleic acids, about 0.5 x $10^5$ different nucleic acids to about 1.0 x $10^5$ different nucleic acids, about 1.0 x $10^5$ different nucleic acids to about 1.0 x $10^9$ different nucleic acids, about 1.0 x $10^5$ different nucleic acids to about 0.5 x $10^9$ different nucleic acids, about 1.0 x $10^5$ different nucleic acids to about 1.0 x $10^8$ different nucleic acids, about 1.0 x $10^5$ different nucleic acids to about 0.5 x $10^8$ different nucleic acids, about 1.0 x $10^5$ different nucleic acids to about 1.0 x $10^7$ different nucleic acids, about 1.0 x $10^5$ different nucleic acids to about 0.5 x $10^7$ different nucleic acids, about 1.0 x $10^5$ different nucleic acids to about 1.0 x $10^6$ different nucleic acids, about 1.0 x $10^5$ different nucleic acids to about 0.5 x $10^6$ different nucleic acids, about 0.5 x $10^6$ different nucleic acids to about 1.0 x $10^9$ different nucleic acids, about 0.5 x $10^6$ different nucleic acids to about 0.5 x $10^9$ different nucleic acids, about 0.5 x $10^6$ different nucleic acids to about 1.0 x $10^8$ different nucleic acids, about 0.5 x $10^6$ different nucleic acids to about 0.5 x $10^8$ different nucleic acids, about 0.5 x $10^6$ different nucleic acids to about 1.0 x $10^7$ different nucleic acids, about 0.5 x $10^6$ different nucleic acids to about 0.5 x $10^7$ different nucleic acids, about 0.5 x $10^6$ different nucleic acids to about 1.0 x $10^6$ different nucleic acids, about 1.0 x $10^6$ different nucleic acids to about 1.0 x $10^9$ different nucleic acids, about 1.0 x $10^6$ different nucleic acids to about 0.5 x $10^9$ different nucleic acids, about 1.0 x $10^6$ different nucleic acids to about 1.0 x $10^8$ different nucleic acids, about 1.0 x $10^6$ different nucleic acids to about 0.5 x $10^8$ different nucleic acids, about 1.0 x $10^6$ different nucleic acids to about 1.0 x $10^7$ different nucleic acids, about 1.0 x $10^6$ different nucleic acids to about 0.5 x $10^7$ different nucleic acids, about 0.5 x $10^7$ different nucleic acids to about 1.0 x $10^9$ different nucleic acids, about 0.5 x $10^7$ different nucleic acids to about 0.5 x $10^9$ different nucleic acids, about 0.5 x $10^7$ different nucleic acids to about 1.0 x $10^8$ different nucleic acids, about 0.5 x $10^7$ different nucleic acids to about 0.5 x $10^8$ different nucleic acids, about 0.5 x $10^7$ different nucleic acids to about 1.0 x $10^7$ different nucleic acids, about 1.0 x $10^7$ different nucleic acids to about 1.0 x $10^9$ different nucleic acids, about 1.0 x $10^7$ different nucleic acids to about 0.5 x $10^9$ different nucleic acids, about 1.0 x $10^7$ different nucleic acids to about 1.0 x $10^8$ different nucleic acids, about 1.0 x $10^7$ different nucleic acids to about 0.5 x $10^8$ different nucleic acids, about 0.5 x $10^8$ different nucleic acids to about 1.0 x $10^9$ different nucleic acids, about 0.5 x $10^8$ different nucleic acids to about 0.5 x $10^9$ different nucleic acids, about 0.5 x $10^8$ different nucleic acids to about 1.0 x $10^8$ different nucleic acids, about 1.0 x $10^8$ different nucleic acids to about 1.0 x $10^9$ different nucleic acids, about 1.0 x $10^8$ different nucleic acids to about 0.5 x $10^9$ different nucleic acids, or about 0.5 x $10^9$ different nucleic acids to about 1.0 x $10^9$ different nucleic acids.

In some embodiments of any of the methods described herein, a nucleic acid that is present in a library (e.g., and that is captured by the methods described herein) can include or consist of a sequence that has a high GC content. In some embodiments, the GC content of a nucleic acid in a library or a portion thereof (e.g., a target oligonucleotide sequence present in a nucleic acid in a library) can have a GC percentage of about 60% and above (e.g., about 62% and above, about 64% and above, about 65% and above, about 68% and above, about 70% and above, about 72% and above, about 74% and above, about 75% and above, about 78% and above, about 80% and above, about 82% and above, about 84% and above, about 85% and above, about 88% and above, about 90% and above, about 92% and above, about 94% and above, about 95% and above, or about 98% and above, or about 60%, about 61%, about 62%, about 63%, about 64%, about 65%, about 66%, about 67%, about 68%, about 69%, about 70%, about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, or 100%).

In some embodiments of any of the methods described herein, a nucleic acid that is present in a library (e.g., and that is captured by the methods described herein) can include or consist of a sequence that has a low GC content. In some embodiments, the GC content of a nucleic acid in a library or a portion thereof (e.g., a target oligonucleotide sequence present in a nucleic acid in a library) can have a GC percentage of about 59% and below (e.g., about 58% and below, about 56% and below, about 54% and below, about 52% and below, about 50% and below, about 48% and below, about 46% and below, about 44% and below, about 42% and below, about 40% and below, about 38% and below, about 36% and below, about 34% and below, about 32% and below, about 30% and below, about 28% and below, about 26% and below, about 24% and below, about 22% and below, about 20% and below, about 18% and below, about 16% and below, about 14% and below, about 12% and below, about 10% and below, about 8% and below, about 6% and below, about 4% and below, about 2% and below, or about 1% and below, or about 0.5%, about 1%, about 2%, about 3%, about 4%, about 5%, about 6%, about 7%, about 8%, about 9%, about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%, about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, or about 59%).

Some embodiments of any of the methods described herein further include generating a library comprising the steps of: fragmenting double-stranded DNA (e.g., genomic DNA or cellular DNA from a mammalian cell, e.g., mammalian cells present in a biopsy sample), performing end repair and dA-tailing, ligating an adaptor, and performing PCR amplification, thus yielding a library. Additional methods for generating a library are known in the art.

Probes

The methods describe herein include the use of a single-stranded probe that includes a sequence that is complementary to a target oligonucleotide sequence (e.g., any of the target sequences described herein).

In some embodiments of any of the methods described herein, the probe contains a total of about 10 nucleotides (nt) to about 800 nts, about 10 nt to about 500 nt, about 10 nt to about 250 nt, about 10 nt to about 100 nt, about 10 nt to about 50 nt, about 10 nt to about 40 nt, about 10 nt to about 30 nt, about 10 nt to about 20 nt, about 10 nt to about 15 nt, about 20 nt to about 800 nt, about 20 nt to about 500 nt, about 20 nt to about 200 nt, about 20 nt to about 100 nt, about 20 nt to about 50 nt, about 20 nt to about 40 nt, about 50 nt to about 800 nt, about 50 nt to about 500 nt, about 50 nt to about 250 nt, about 50 nt to about 100 nt, about 100 nt to about 800 nt, about 100 nt to about 500 nt, about 100 nt to about 250 nt, about 100 nt to about 200 nt, about 150 nt to about 800 nt, about 150 nt to about 500 nt, about 150 nt to about 250 nt, about 150 nt to about 200 nt, about 200 nt to about 800 nt, about 200 nt to about 500 nt, about 200 nt to about 400 nt, about 200 nt to about 300 nt, about 200 nt to about 250 nt, about 250 nt to about 500 nt, about 500 nt to about 800 nt,.

In some embodiments, the sequence that is complementary to a target oligonucleotide sequence and/or the target oligonucleotide sequence include a total of about 8 nucleotides (nt) to about 400 nt, about 8 nt to about 200 nt, about 8 nt to about 100 nt, about 8 nt to about 50 nt, about 8 nt to about 30 nt, about 8 nt to about 20 nt, about 8 nt to about 16 nt, about 8 nt to about 10 nt, about 10 nt to about 400 nt, about 10 nt to about 200 nt, about 10 nt to about 100 nt, about 10 nt to about 50 nt, about 10 nt to about 20 nt, about 20 nt to about 400 nt, about 20 nt to about 200 nt, about 20 nt to about 100 nt, about 50 nt to about 400 nt, about 50 nt to about 100 nt, about 75 nt to about 400 nt, about 75 nt to about 200 nt, about 75 nt to about 100 nt, about 100 nt to about 400 nt, about 100 nt to about 200 nt, about 200 nt to about 400 nt, about 200 nt to about 300 nt, about 300 nt to about 400 nt.

In some embodiments of any of the methods described herein, the sequence that is complementary to a target oligonucleotide sequence and/or the target oligonucleotide sequence can include or consist of a sequence that has a high GC content. In some embodiments, the GC content of the sequence that is complementary to a target oligonucleotide sequence and/or the target oligonucleotide sequence can have a GC percentage of about 60% and above (e.g., about 62% and above, about 64% and above, about 65% and above, about 68% and above, about 70% and above, about 72% and above, about 74% and above, about 75% and above, about 78% and above, about 80% and above, about 82% and above, about 84% and above, about 85% and above, about 88% and above, about 90% and above, about 92% and above, about 94% and above, about 95% and above, or about 98% and above, or about 60%, about 61%, about 62%, about 63%, about 64%, about 65%, about 66%, about 67%, about 68%, about 69%, about 70%, about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, or 100%).

In some embodiments of any of the methods described herein, the sequence that is complementary to a target oligonucleotide sequence and/or the target oligonucleotide sequence can include or consist of a sequence that has a low GC content. In some embodiments, the GC content of a sequence that is complementary to a target oligonucleotide sequence and/or the target oligonucleotide sequence can have a GC percentage of about 59% and below (e.g., about 58% and below, about 56% and below, about 54% and below, about 52% and below, about 50% and below, about 48% and below, about 46% and below, about 44% and below, about 42% and below, about 40% and below, about 38% and below, about 36% and below, about 34% and below, about 32% and below, about 30% and below, about 28% and below, about 26% and below, about 24% and below, about 22% and below, about 20% and below, about 18% and below, about 16% and below, about 14% and below, about 12% and below, about 10% and below, about 8% and below, about 6% and below, about 4% and below, about 2% and below, or about 1% and below, or about 0.5%, about 1%, about 2%, about 3%, about 4%, about 5%, about 6%, about 7%, about 8%, about 9%, about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%, about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, or about 59%).

The choice of exons targeted by the probes is primarily driven by National Comprehensive Cancer Network (NCCN) guidelines to cover actionable variants. Furthermore, to have high hybridization efficiency, the probes are designed to have the coverage to panel size ratio as high as possible. This is done by selecting exons that harbor most cancer variants based on The Cancer Genome Atlas (TGCA). Further selection is done by designing probes complementary to exons that cover maximum number of variants over large population of patients based on data from Catalog of Somatic Mutations Cancer (COSMIC). This way, the number of probes are small yet offer broad coverage thereby improving hybridization efficiency.

In order to improve coverage uniformity, the probes include extra nucleotides flanking the covered exons thereby covering exon-intron splice junction. This results in covering the entirety of the exon uniformly without any exon dropouts.

In some examples, the target oligonucleotide sequence is a sequence from a protooncogene. In some examples, the target oligonucleotide sequence is a sequence from an oncogene. In some examples, the target oligonucleotide sequence is an oncogenic kinase fusion protein. In some examples, the target oligonucleotide sequence is a sequence of a gene listed in Table 1.

**Blocking**

In order to reduce non-specific binding by preventing amplification of dominant and/or unwanted DNA templates and improve on-target rate, a blocking step is performed. Various examples of blocking oligonucleotides are known in the art. See, e.g., Xie et al. (2016) Mol. Genet. Genomic Med 4(3): 262-272; Blumenstiel et al. (2010) Curr. Protoc. Hum. Genet. Chapter 18: Unit 18.4; and Vestheim et al. (2011) Methods Mol. Biol. 687: 265-274. For example, blocking oligonucleotides can be obtained from Integrated DNA Technologies (IDT). In some instances, blocking oligonucleotides are combined with salmon sperm DNA and human cot-1 DNA. This constitutes a blocking mixture. In some instances, the blocking mixture is added to the pooled libraries prior to the hybridization step.

**Hybridization**

In the context of this invention, hybridization means hydrogen bonding, which may be Watson-Crick, Hoogsteen, or reversed Hoogsteen hydrogen bonding, between complementary nucleoside or nucleotide bases. For example, adenine and thymine are complementary nucleobases which pair through the formation of hydrogen bonds. Complementary, as used herein, refers to the capacity for precise pairing between two nucleotides. For example, if a nucleotide at a certain position of a probe is capable of hydrogen bonding with a nucleotide at the corresponding position of a target DNA molecule or a target RNA molecule, then the probe and the target DNA molecule or target RNA molecule are considered to be complementary to each other at that position. The probe and the target DNA molecule or target RNA molecule are complementary to each other when a sufficient number of corresponding positions in each molecule are occupied by nucleotides which can hydrogen bond with each other. Thus, "specifically hybridizable" and "complementary" are terms whose use in part indicates a sufficient degree of complementarity or precise pairing such that stable and specific binding occurs between the probe and the target DNA molecule or target RNA molecule. For example, if a base at one position of a probe is capable of hydrogen bonding with a base at the corresponding position of a target DNA molecule or a target RNA molecule, then the bases are considered to be complementary to each other at that position. 100% complementarity is not required.

It is understood in the art that a nucleic acid sequence need not be 100% complementary to that of its target nucleic acid to be specifically hybridizable. A complementary nucleic acid sequence for purposes of the present methods is specifically hybridizable when there is a sufficient degree of complementarity to avoid non-specific binding of the sequence to non-target DNA and/or RNA sequences under conditions in which specific binding is desired, e.g., under conditions in which the *in vitro* assays are performed under suitable conditions of stringency. In some embodiments, a complementary nucleic acid sequence is not complementary to other sequences.

Hybridization techniques are well known to those skilled in the art and are described, for example, in Benton and Davis (Science 196:180, 1977); Grunstein and Hogness (Proc. Natl. Acad. Sci. U.S.A. 72:3961, 1975); Ausubel et al. (Current Protocols in Molecular Biology, Wiley Interscience, New York, 2001); Berger and Kimmel (Guide to Molecular Cloning Techniques, 1987, Academic Press, New York); and Sambrook et al., Molecular Cloning: A Laboratory Manual, Cold Spring Harbor Laboratory Press, New York.

**Methods of capturing an oligonucleotide**

Provided herein are methods of capturing a nucleic acid including a target oligonucleotide sequence that include: contacting a library of nucleic acids including a nucleic acid that includes a target oligonucleotide sequence with a probe that includes a sequence that is complementary to the target oligonucleotide sequence, where the contacting is performed in a tetramethylammonium chloride (TMAC)-based buffer at a temperature of about 60 °C to about 70 °C, and the contacting results in the hybridization of the target oligonucleotide sequence to the sequence that is complementary to the target oligonucleotide sequence, to thereby generate a hybridization product; and isolating the hybridization product from the library.

In some embodiments of any of the methods described herein, the hybridization product is a RNA-DNA product or a DNA-DNA product. In some embodiments, the hybridization product is a RNA-DNA product. In some embodiments, the hybridization product is a DNA-DNA product.

**Contacting a Library of Nucleic Acids**

In some embodiments of any of the methods provided herein, the contacting of a library of nucleic acids including a nucleic acid including a target oligonucleotide sequence with a probe including a sequence that is complementary to the target oligonucleotide sequence is performed in a TMAC-based buffer at a temperature of about 60 °C to about 70 °C (e.g., about 60 °C to about 66 °C, about 60 °C to about 64 °C, about 64 °C to about 68 °C, about 64 °C to about 66 °C, about 64 °C, about 65 °C, about 66 °C, about 67 °C, about 68 °C, about 69 °C, or about 70 °C).

In some embodiments of any of the methods provided herein, the TMAC-based buffer comprises about 0.5 M to about 8.0 M of TMAC (e.g., about 0.5 M to about 6.0 M, about 0.5 M to about 5.5 M, about 0.5 M to about 5.0 M, about 0.5 M to about 4.5 M, about 0.5 M to about 4.0 M, about 0.5 M to about 3.0 M, about 0.5 M to about 2.5 M, about 0.5 M to about 2.0 M, about 1.0 M to about 8.0 M, about 1.0 M to about 6.0 M, about 1.0 M to about 5.5 M, about 1.0 M to about 5.0 M, about 1.0 M to about 4.5 M, about 1.0 M to about 4.0 M, about 1.0 M to about 3.5 M, about 1.0 M to about 3.0 M, about 1.0 M to about 2.5 M, about 1.0 M to about 2.0 M, about 2.0 M to about 8.0 M, about 2.0 M to about 6.0 M, about 2.0 M to about 5.5 M, about 2.0 M to about 5.0 M, about 2.0 M to about 4.5 M, about 2.0 M to about 4.0 M, about 2.5 M to about 8.0 M, about 2.5 M to about 6.0 M, about 2.5 M to about 5.5 M, about 2.5 M to about 5.0 M, about 3.0 M to about 8.0 M, about 3.0 M to about 6.0 M, about 3.0 M to about 5.5 M, about 3.0 M to about 5.0 M, about 3.0 M to about 4.5 M, about 3.0 M to about 4.0 M, about 4.0 M to about 8.0 M, about 4.0 M to about 6.0 M, about 4.0 M to about 5.5 M, about 4.0 M to about 5.0 M, about 4.0 M to about 4.5 M, about 5.0 M to about 8.0 M, about 5.0 M to about 6.0 M, about 5.0 M to about 5.5 M, about 6.0 M to about 8.0 M, about 7.0 M to about 8.0 M, about 7.0 M to about 7.5 M, about 7.5 M to about 8.0 M, or about 0.5 M, about 0.6 M, about 0.7 M, about 0.8 M, about 0.9 M, about 1.0 M, about 1.2 M, about 1.4 M, about 1.6 M, about 1.8 M, about 2.0 M, about 2.2 M, about 2.4 M, about 2.6 M, about 2.7 M, about 2.8 M, about 3.0 M, about 3.2 M, about 3.4 M, about 3.6 M, about 3.8 M, about 4.0 M, about 4.2 M, about 4.4 M, about 4.6 M, about 4.8 M, about 5.0 M, about 5.2 M, about 5.4 M, about 5.6 M, about 5.8 M, about 6.0 M, about 6.2 M, about 6.4 M, about 6.6 M, about 6.8 M, about 7.0 M, about 7.2 M, about 7.4 M, about 7.6 M, about 7.8 M, or about 8.0 M).

In some embodiments of any of the methods described herein, the TMAC-based buffer further includes one or more (two, three, four, or five) of:

about 10 mM to about 200 mM 2-amino-2-(hydroxymethyl)propane-1,3-diol (Tris) (e.g., about 10 mM to about 150 mM, about 10 mM to about 100 mM, about 10 to about 50 mM, about 20 mM to about 200 mM, about 20 mM to about 150 mM, about 20 to about 100 mM, about 20 to about 60 mM, about 20 to about 50 mM, about 30 mM to about 200 mM, about 30 mM to about 150 mM, about 30 to about 100 mM, about 30 to about 60 mM, about 30 to about 50 mM, about 40 mM to about 200 mM, about 40 mM to about 150 mM,, about 40 to about 60 mM, about 40 to about 50 mM, about 50 mM to about 200 mM, about 50 mM to about 150 mM, about 50 to about 100 mM, about 90 mM to about 200 mM, about 90 mM to about 150 mM, about 100 mM to about 200 mM, about 100 mM to about 150 mM,, or about 10 mM, about 20 mM, about 30 mM, about 40 mM, about 50 mM, about 60 mM, about 70 mM, about 80 mM, about 90 mM, about 100 mM, about 150 mM, about 160 mM, about 170 mM, about 180 mM about 190 mM, about 200 mM Tris) at pH 6.- 8.0 (e.g., pH 6.0-7.5, pH 6.0-7.0, pH 6.0-6.5, pH 6.5-8.0, pH 6.5-7.5, pH 6.5-7..0, pH 7.0-8.0, pH 7.0-7.5, pH 6.0, pH 6.5, pH 7.0, pH 7.5, pH 8.0);
about 1X to about 5X of Denhardt's Solution (e.g., about 1X to about 4X, about 1X to about 3X, about 1X to about 2X, about 2X to about 5X, about 2X to about 4X, about 2X to about 3X, about 3X to about 5X, about 3X to about 4X, or about 4X to about 5X, or about 1X, about 1.5X, about 2X, about 2.5X, about 3X, about 3.5X, about 4X, about 4.5X, or about 5X Denhardt's Solution);
about 0.01% to about 0.2% of Tween-20 (e.g., about 0.01% to about 0.10%, about 0.01% to about 0.06%, about 0.01% to about 0.05%, about 0.01% to about 0.04%, about 0.02% to about 0.20%, about 0.02% to about 0.10%, about 0.02% to about 0.06%, about 0.02% to about 0.04%, about 0.10% to about 0.20 about 0.10% to about 0.16%, about 0.14% to about 0.20%, about 0.16% to about 0.20%, about 0.01%, about 0.02%, about 0.04%, about 0.06%, about 0.08%, about 0.1%, or about 0.2% Tween-20);
about 0.5 mM to about 15 mM ethylenedioaminetetraacetic acid (EDTA) (e.g., about 0.5 mM to about 12.5 mM, about 0.5 mM to about 12.0 mM, about 0.5 mM to about 10.0 mM, about 0.5 mM to about 8.0 mM, about 0.5 mM to about 7.0 mM, about 0.5 mM to about 6.0 mM, about 0.5 mM to about 5.0 mM, about 1.0 mM to about 15 mM, about 1.0 mM to about 13.5 mM, about 1.0 mM to about 13.0 mM, about 1.0 mM to about 12.5 mM, about 1.0 mM to about 12.0 mM, about 1.0 mM to about 10 mM, about 1.0 mM to about 7.0 mM, about 1.0 mM to about 6.0 mM, about 1.0 mM to about 5.0 mM, about 2.0 mM to about 15 mM, about 2.0 mM to about 13.5 mM, about 2.0 mM to about 13.0 mM, about 2.0 mM to about 12.5 mM, about 2.0 mM to about 12.0 mM, about 2.0 mM to about 10 mM, about 2.0 mM to about 8.0 mM, about 2.0 mM to about 6.0 mM, about 3.0 mM to about 15 mM, about 3.0 mM to about 14.0 mM, about 3.0 mM to about 13.0 mM, about 3.0 mM to about 10 mM, about 3.0 mM to about 8.0 mM, about 3.0 mM to about 7.0 mM, about 3.0 mM to about 6.5 mM, about 3.0 mM to about 6.0 mM, about 3.0 mM to about 5.5 mM, about 3.0 mM to about 5.0 mM, about 3.0 mM to about 4.0 mM, about 5.0 mM to about 15 mM, about 5.0 mM to about 13.5 mM, about 5.0 mM to about 13.0 mM, about 5.0 mM to about 12.5 mM, about 5.0 mM to about 12.0 mM, about 5.0 mM to about 10 mM, about 5.0 mM to about 8.0 mM, about 5.0 mM to about 6.5 mM, about 5.0 mM to about 6.0 mM, about 6.0 mM to about 15 mM, about 6.0 mM to about 13.0 mM, about 6.0 mM to about 12.5 mM, about 6.0 mM to about 12.0 mM, about 6.0 mM to about 10 mM, about 8.0 mM to about 15 mM, about 8.0 mM to about 13.0 mM, about 8.0 mM to about 12.5 mM, about 8.0 mM to about 12.0 mM, about 8.0 mM to about 10 mM, about 10.0 mM to about 15 mM, about 10.0 mM to about 13.0 mM, about 10.0 mM to about 12.5 mM, about 10.0 mM to about 12.0 mM, or about 11.0 mM to about 13.0 mM, about 5 mM, about 6 mM, about 8 mM, about 10 mM, about 12 mM, about 14 mM, or about 15 mM); and

about 0.5% to about 25% (v/v) formamide (e.g., about 0.5% (v/v) to about 20.0% (v/v), about 0.5% (v/v) to about 15.0% (v/v), about 0.5% (v/v) to about 10.0% (v/v), about 0.5% (v/v) to about 5.0% (v/v), about 0.5% (v/v) to about 3.0% (v/v), about 0.5% (v/v) to about 2.0% (v/v), about 0.5% (v/v) to about 1.0% (v/v), about 1.0% (v/v) to about 25% (v/v), about 1.0% (v/v) to about 20.0% (v/v), about 1.0% (v/v) to about 15.0% (v/v), about 1.0% (v/v) to about 12.5% (v/v), about 1.0% (v/v) to about 10.0% (v/v), \about 1.0% (v/v) to about 5.0% (v/v), about 5.0% (v/v) to about 25% (v/v), about 5.0% (v/v) to about 20.0% (v/v), about 5.0% (v/v) to about 15.0% (v/v), about 5.0% (v/v) to about 10.0% (v/v), about 10.0% (v/v) to about 25% (v/v), about 10.0% (v/v) to about 20.0% (v/v), about 10.0% (v/v) to about 15.0% (v/v), about 10.0% (v/v) to about 12.5% (v/v), about 12.5% (v/v) to about 25% (v/v), about 12.5% (v/v) to about 20.0% (v/v), about 15.0% (v/v) to about 25% (v/v), about 15.0% (v/v) to about 20.0% (v/v), about 17.5% (v/v) to about 25% (v/v), about 17.5% (v/v) to about 22.5% (v/v), about 17.5% (v/v) to about 20.0% (v/v), or about 20.0% (v/v) to about 25% (v/v) formamide).

In some embodiments, the TMAC-based buffer includes about 10 mM to about 200 mM (or any of the subranges of this range described herein) of 2-amino-2-(hydroxymethyl)propane-1,3-diol (Tris); about 1X to about 5X (or any of the subranges of this range described herein) Denhardt's Solution; about 0.01% to about 0.2% (or any of the subranges of this range described herein) Tween-20; about 0.5 mM to about 10 mM (or any of the subranges of this range described herein) ethylenedioaminetetraacetic acid (EDTA); and about 0.5% to about 25% (v/v) (or any of the subranges of this range described herein) formamide.

In some embodiments, the TMAC-based buffer includes about 40 mM to about 60 mM (or any of the subranges of this range described herein) 2-amino-2-(hydroxymethyl)propane-1,3-diol (Tris); about 2X to about 3X (or any of the subranges of this range described herein) Denhardt's Solution; about 0.01% to about 0.05% (or any of the subranges of this range described herein) Tween-20; about 0.5 mM to about 7 mM (or any of the subranges of this range described herein) ethylenedioaminetetraacetic acid (EDTA); and about 0.55% (v/v) to about 25% (v/v) (or any of the subranges of this range described herein) formamide.

In some embodiments, the TMAC-based buffer includes about 2.7 M TMAC, about 50 mM Tris (pH 8.0), about 2.5X Denhardt's Solution, about 0.010% Tween-20, about 6 mM EDTA, and about 20% formamide.

In some embodiments, the TMAC-based buffer includes about 5.4 M TMAC, about 100 mM Tris (pH 8.0), about 5X Denhardt's Solution, about 0.02% Tween-20, about 12 mM EDTA.

As described herein, 1X Denhardt's Solution is 1% Ficoll, 1% polyvinylpyrrolidone, and 1% bovine serum albumin.

In some embodiments of any of the methods described herein, the contacting step is performed for about 1 hour to about 48 hours (e.g., about 1 hour to about 36 hours, about 1 hour to about 24 hours, about 1 hour to about 18 hours, about 1 hour to about 16 hours, about 1 hours to about 14 hours, about 1 hour to about 12 hours, about 1 hour to about 10 hours, about 1 hour to about 6 hours, about 1 hour to about 2 hours; about 4 hours to about 48 hours, about 4 hours to about 24 hours, about 4 hours to about 16 hours, about 4 hours to about 12 hours, about 4 hours to about 10 hours, about 4 hours to about 8 hours, about 4 hours to about 6 hours, about 6 hours to about 14 hours, about 6 hours to about 12 hours, about 6 hours to about 10 hours, about 10 hours to about 48 hours, about 10 hours to about 24 hours, about 10 hours to about 20 hours, about 10 hours to about 18 hours, about 10 hours to about 16 hours, about 10 hours to about 14 hours, about 10 hours to about 12 hours, about 12 hours to about 48 hours, about 12 hours to about 24 hours, about 12 hours to about 18 hours, about 12 hours to about 16 hours, about 16 hours to about 48 hours, about 16 hours to about 24 hours, about 16 hours to about 18 hours, about 20 hours to about 48 hours, or about 20 hours to about 24 hours, about 24 hours to about 48 hours).

**Bead Binding Step**

The probes are biotinylated and after hybridizing with targeted nucleic acid regions form biotinylated hybrids.

In order to separate the targeted nucleic acids from non-targeted nucleic acids, the biotinylated hybrids are allowed to bind to Streptavidin coated magnetic beads. The binding occurs in up to 2.0 M salt (e.g., about 0.1 M to about 2.0 M, about 0.1 M to about 1.0 M, about 0.1 M to about 0.5 M, about 1.0 M to about 2.0 M, about 1.0 M to about 1.5 M, about 0.2 M, about 0.3 M, about 0.4 M, about 0.5 M, about 0.6 M, about 0.7 M, about 0.8 M, about 0.9 M, about 1.0 M, about 1.2 M, about 1.4 M, about 1.6 M, about 1.8 M, or about 2.0 M).in presence of about 0.01 M to about 0.1 M Tris ph8.0 (e.g., about 0.01 M to about 0.08 M, about 0.01 M to about 0.06 M, about 0.01 M to about 0.04 M, about 0.01 M to about 0.02 M, about 0.02 M to about 0.1 M, about 0.02 M to about 0.08 M, about 0.02 M to about 0.06 M, about 0.02 M to about 0.04 M, about 0.04 M to about 0.1 M, about 0.04 M to about 0.08 M, about 0.04 M to about 0.06 M, about 0.06 M to about 0.1 M, about 0.06 M to about 0.08 M, about 0.01 M, about 0.02 M, about 0.04 M, about 0.06 M, about 0.08 M, about 0.1 M) and about 0.1 M to about 1.0 M EDTA (e.g., about 0.1 M to about 0.8 M, about 0.1 M to about 0.6 M, about 0.1 M to about 0.5 M, about 0.1 M to about 0.4 M, about 0.1 M to about 0.2 M, about 0.2 M to about 1.0 M, about 0.2 M to about 0.8 M, about 0.2 M to about 0.6 M, about 0.2 M to about 0.5 M, about 0.2 M to about 0.4 M, about 0.4 M to about 1.0 M, about 0.4 M to about 0.8 M, about 0.4 M to about 0.6 M, about 0.4 M to about 0.5 M, about 0.5 M to about 1.0 M, about 0.5 M to about 0.8 M, about 0.5 M to about 0.6 M, about 0.6 M to about 1.0 M, about 0.6 M to about 0.8 M, about 0.1 M, about 0.2 M, about 0.4 M, about 0.5 M, about 0.6 M, about 0.8 M or about 1.0 M). The binding is carried out at a temperature of about 16 °C to about 65 °C (e.g., about 16 °C to about 60 °C, about 16 °C to about 50 °C, about 16 °C to about 42 °C, about 16 °C to about 40 °C, about 16 °C to about 38 °C, about 16 °C to about 36 °C, about 16 °C to about 30 °C, about 16 °C to about 28 °C, about 16 °C to about 24 °C, about 16 °C to about 20 °C, about 16 °C to about 18 °C, about 20 °C to about 65 °C, about 20 °C to about 42 °C, about 20 °C to about 32 °C, about 20 °C to about 26 °C, about 20 °C to about 24 °C, about 20 °C to about 22 °C, about 24 °C to about 65 °C, about 24 °C to about 60 °C, about 24 °C to about 42 °C, about 24 °C to about 40 °C, about 24 °C to about 38 °C, about 24 °C to about 36 °C, about 24 °C to about 34 °C, about 24 °C to about 32 °C, about 24 °C to about 30 °C, about 24 °C to about 28 °C, about 24 °C to about 26 °C, about 26 °C to about 65 °C, about 26 °C to about 38 °C, about 30 °C to about 65 °C, about 30 °C to about 42 °C, about 30 °C to about 34 °C, about 32 °C to about 38 °C, about 32 °C to about 36 °C, about 36 °C to about 65 °C, about 36 °C to about 48 °C, about 40 °C to about 65 °C, about 40 °C to about 44 °C, about 50 °C to about 65 °C, about 56 °C to about 65 °C, about 60 °C to about 65 °C, about 16 °C, about 22 °C, about 24 °C, about 26 °C, about 28 °C, about 30 °C, about 32 °C, about 34 °C, about 36 °C, about 38 °C, about 40 °C, about 42 °C, about 44 °C, about 46 °C, about 48 °C, about 50 °C, about 52 °C, about 54 °C, about 56 °C, about 58 °C, about 60 °C, about 62 °C, about 64 °C, or about 65 °C) for about 10 minutes to about 60 minutes (e.g., about 10 minutes to about 50 minutes, about 10 minutes to about 45 minutes, about 10 minutes to about 40 minutes, about 10 minutes to about 30 minutes, about 10 minutes to about 20 minutes, about 10 minutes to about 15 minutes, about 15 minutes to about 60 minutes, about 15 minutes to about 45 minutes, about 15 minutes to about 30 minutes, about 20 minutes to about 60 minutes, about 20 minutes to about 45 minutes, about 20 minutes to about 30 minutes, about 20 minutes to about 25 minutes, about 25 minutes to about 60 minutes, about 25 minutes to about 30 minutes, about 30 minutes to about 60 minutes, about 30 minutes to about 45 minutes, about 45 minutes to about 60 minutes, about 10 minutes, about 15 minutes, about 20 minutes, about 25 minutes, about 30 minutes, about 35 minutes, about 40 minutes, about 45 minutes, about 50 minutes, about 55 minutes, or about 60 minutes) in a volume of about 20 μL to about 500 μL (e.g., about 20 μL to about 400 μL, about 20 μL to about 300 μL, about 20 μL to about 250 μL, about 20 μL to about 200 μL, about 20 μL to about 100 μL, about 20 μL to about 50 μL, about 50 μL to about 500 μL, about 50 μL to about 250 μL, about 50 μL to about 100 μL, about 100 μL to about 500 μL, about 100 μL to about 400 μL, about 100 μL to about 300 μL, about 100 μL to about 250 μL, about 100 μL to about 200 μL, about 100 μL to about 150 μL, about 150 μL to about 500 μL, about 150 μL to about 300 μL, about 150 μL to about 200 μL, about 200 μL to about 500 μL, about 200 μL to about 400 μL, about 200 μL to about 250 μL, about 250 μL to about 500 μL, about 300 μL to about 500 μL, about 300 μL to about 400 μL, about 400 μL to about 500 μL, about 25 μL, about 50 μL, about 100 μL, about 150 μL, about 200 μL, about 250 μL, about 300 μL, about 350 μL, about 400 μL, about 450 μL, or about 500 μL).

During the binding step, the biotinylated hybrids of the targeted nucleic acid bind to the magnetic beads and separated from the non-targeted nucleic acids using a magnet. In order to further remove non-targeted non-specifically bound nucleic acids, washing steps are performed. These steps ensure maximum on-target coverage and minimum off-target rates.

**Washing Step**

In some embodiments of any of the methods described herein, the method can further include at least one (e.g., 2, 3, or 4) washing steps after the contacting step and the isolating step.

In some embodiments of any of the methods described herein, the at least one washing step includes the use of a bead wash buffer (e.g., any of the bead wash buffers described herein). In some embodiments, the at least one washing step includes washing uses a bead wash buffer (e.g., any of the exemplary bead wash buffers described herein) at a temperature of about 16 °C to about 30 °C (e.g., about 16 °C to about 24 °C, about 16 °C to about 20 °C, about 16 °C to about 18 °C, about 20 °C to about 30 °C, about 20 °C to about 26 °C, about 20 °C to about 24 °C, about 20 °C to about 22 °C, about 23 °C to about 27 °C, about 24 °C to about 30 °C, about 24 °C to about 28 °C, about 24 °C to about 26 °C, about 26 °C to about 30 °C, about 26 °C to about 28 °C, or about 28 °C to about 30 °C, or about 16 °C, about 18 °C, about 20 °C, about 22 °C, about 24 °C, about 26 °C, about 28 °C, or about 30 °C) for about 1 minute to about 10 hours (e.g. about 1 minute to about 6 hours, about 1 minute to about 4 hours, about 1 minute to about 2 hours, about 1 minute to about 1hour, about 1 minute to about 45 minutes about 1 minute to about 30 minutes, about 1 minute to about 25 minutes, about 1 minute to about 20 minutes, about 1 minute to about 15 minutes, about 1 minute to about 10 minutes, about 1 minute to about 5 minutes, about 5 minutes to about 10 hours, about 5 minutes to about 5 hours, about 5 minutes to about 2 hours, about 5 minutes to about 1 hour, about 5 minutes to about 45 minutes, about 5 minutes to about 40 minutes, about 5 minutes to about 30 minutes, about 5 minutes to about 25 minutes, about 5 minutes to about 20 minutes, about 5 minutes to about 15 minutes, about 5 minutes to about 10 minutes, about 15 minutes to about 10 hours, about 15 minutes to about 8 hours, about 15 minutes to about 6 hours, about 15 minutes to about 4 hours, about 15 minutes to about 2 hours, about 15 minutes to about 1 hour, about 15 minutes to about 45 minutes, about 15 minutes to about 30 minutes, about 15 minutes to about 25 minutes, about 15 minutes to about 20 minutes, about 30 minutes to about 10 hours, about 30 minutes to about 4 hours, about 30 minutes to about 2 hours, about 30 minutes to about 1 hour, about 30 minutes to about 45 minutes, about 45 minutes to about 10 hours, about 45 minutes to about 6 hours, about 45 minutes to about 4 hours, about 45 minutes to about 1 hour, about 1 hour to about 10 hours, about 1 hour to about 8 hours, about 1 hour to about 6 hours, about 1 hour to about 5 hours, about 1 hour to about 4 hours, about 1 hour to about 2 hours, about 2 hours to about 10 hours, about 2 hours to about 8 hours, about 2 hours to about 6 hours, , about 2 hours to about 4 hours, about 4 hours to about 10 hours, about 4 hours to about 8 hours, about 4 hours to about 6 hours, about 5 hours to about 10 hours, about 5 hours to about 6 hours, or about 6 hours to about 10 hours) in a volume of about 20 μL to about 500 μL (e.g., about 20 μL to about 400 μL, about 20 μL to about 300 μL, about 20 μL to about 250 μL, about 20 μL to about 200 μL, about 20 μL to about 100 μL, about 20 μL to about 50 μL, about 50 μL to about 500 μL, about 50 μL to about 250 μL, about 50 μL to about 100 μL, about 100 μL to about 500 μL, about 100 μL to about 400 μL, about 100 μL to about 300 μL, about 100 μL to about 250 μL, about 100 μL to about 200 μL, about 100 μL to about 150 μL, about 150 μL to about 500 μL, about 150 μL to about 300 μL, about 150 μL to about 200 μL, about 200 μL to about 500 μL, about 200 μL to about 400 μL, about 200 μL to about 250 μL, about 250 μL to about 500 μL, about 300 μL to about 500 μL, about 300 μL to about 400 μL, about 400 μL to about 500 μL, about 25 μL, about 50 μL, about 100 μL, about 150 μL, about 200 μL, about 250 μL, about 300 μL, about 350 μL, about 400 μL, about 450 μL).

In some embodiments, the bead wash buffer includes about 1 M to about 10 M sodium chloride (NaCl) (e.g., about 1 M to about 8 M, about 1 M to about 6 M, about 1 M to about 5 M, about 1 M to about 4 M, about 1 M to about 3 M, about 1 M to about 2 M, about 2 M to about 10 M, about 2 M to about 8 M, about 2 M to about 6 M, about 2 M to about 5 M, about 2 M to about 4 M, about 4 M to about 10 M, about 4 M to about 8 M, about 4 M to about 6 M, about 4 M to about 5 M, about 5 M to about 10 M, about 6 M to about 8 M, about 8 M to about 10 M, or about 1M, about 2 M, about 3 M, about 4 M, about 5 M, about 6 M, about 7 M, about 8 M, about 9 M, about 10 M), about 0.5 M to about 5 M Tris-Cl (pH 8.0) (e.g., about 0.5 M to about 4.0 M, about 0.5 M to about 3.0 M, about 0.5 M to about 2.5 M, about 0.5 M to about 2.0 M, about 0.5 M to about 1.0 M, about 1.0 M, to about 5.0 M, about 1.0 M to about 4.0 M, about 1.0 M to about 2.5 M, about 1.0 M to about 2.0 M, about 1.5 M to about 5.0 M, about 1.5 M to about 4.0 M, about 1.5 M to about 3.0 M, about 1.5 M to about 2.5 M, about 1.5 M to about 2.0 M, about 2.0 M to about 5.0 M, about 2.0 M to about 4.0 M, about 2.0 M to about 3.0, about 2.5 M to about 5.0 M, about 3.0 M to about 5.0 M, about 4.0 M to about 5.0 M, about 1.0 M, about 1.5 M, about 2.0 M, about 2.5 M, about 3.0 M, about 3.5 M, about 4.0 M, about 4.5 M, or about 5.0 M), and about 0.1 M to about 1.0 M EDTA (e.g., about 0.1 M to about 0.5 M, about 0.1 M to about 0.2 M, about 0.2 M to about 1.0 M, about 0.2 M to about 0.5 M, about 0.4 M to about 0.8 M, about 0.4 M to about 0.6 M, about 0.4 M to about 0.5 M, about 0.5 M to about 1.0 M, about 0.1 M, about 0.2 M, about 0.4 M, about 0.5 M, about 0.6 M, about 0.8 M or about 1.0 M EDTA).

In some embodiments of any of the methods described herein, the at least one washing step includes the use of a low stringency buffer (e.g., any of the exemplary low stringency buffers described herein) and a high stringency buffer (e.g., any of the exemplary high stringency buffers described herein). In some embodiments, the at least one washing step includes washing uses a low stringency buffer (e.g., any of the exemplary low stringency buffers described herein) at a temperature of about 16 °C to about 30 °C (e.g., about 16 °C to about 24 °C, about 16 °C to about 20 °C, about 16 °C to about 18 °C, about 18 °C to about 30 °C, about 18 °C to about 24 °C, about 18 °C to about 20 °C, about 20 °C to about 30 °C, about 20 °C to about 24 °C, about 20 °C to about 22 °C, about 23 °C to about 27 °C, about 24 °C to about 30 °C, about 24 °C to about 28 °C, about 24 °C to about 26 °C, about 26 °C to about 30 °C, about 26 °C to about 28 °C, or about 28 °C to about 30 °C, or about 16 °C, about 18 °C, about 20 °C, about 22 °C, about 24 °C, about 26 °C, about 28 °C, or about 30 °C) for about 1 minute to about 10 hours (e.g., about 1 minute to about 6 hours, about 1 minute to about 4 hours, about 1 minute to about 2 hours, about 1 minute to about 1 hour, about 1 minute to about 45 minutes about 1 minute to about 30 minutes, about 1 minute to about 25 minutes, about 1 minute to about 20 minutes, about 1 minute to about 15 minutes, about 1 minute to about 10 minutes, about 1 minute to about 5 minutes, about 5 minutes to about 10 hours, about 5 minutes to about 5 hours, about 5 minutes to about 2 hours, about 5 minutes to about 1 hour, about 5 minutes to about 45 minutes, about 5 minutes to about 40 minutes, about 5 minutes to about 30 minutes, about 5 minutes to about 25 minutes, about 5 minutes to about 20 minutes, about 5 minutes to about 15 minutes, about 5 minutes to about 10 minutes, about 15 minutes to about 10 hours, about 15 minutes to about 8 hours, about 15 minutes to about 6 hours, about 15 minutes to about 4 hours, about 15 minutes to about 2 hours, about 15 minutes to about 1 hour, about 15 minutes to about 45 minutes, about 15 minutes to about 30 minutes, about 15 minutes to about 25 minutes, about 15 minutes to about 20 minutes, about 30 minutes to about 10 hours, about 30 minutes to about 4 hours, about 30 minutes to about 2 hours, about 30 minutes to about 1 hour, about 30 minutes to about 45 minutes, about 45 minutes to about 10 hours, about 45 minutes to about 6 hours, about 45 minutes to about 4 hours, about 45 minutes to about 1 hour, about 1 hour to about 10 hours, about 1 hour to about 8 hours, about 1 hour to about 6 hours, about 1 hour to about 5 hours, about 1 hour to about 4 hours, about 1 hour to about 2 hours, about 2 hours to about 10 hours, about 2 hours to about 8 hours, about 2 hours to about 6 hours, , about 2 hours to about 4 hours, about 4 hours to about 10 hours, about 4 hours to about 8 hours, about 4 hours to about 6 hours, about 5 hours to about 10 hours, about 5 hours to about 6 hours, or about 6 hours to about 10 hours).

In some embodiments, the low stringency buffer includes a buffered solution (e.g., a buffered saline solution) and optionally, a detergent. In some embodiments, the low stringency buffer includes saline-sodium citrate (SSC) buffer and optionally, further includes a detergent (e.g., sodium dodecyl sulfate (SDS)). In some embodiments, the low stringency buffer includes about 0.5X to about 2.5X SSC (e.g., about 0.5X to about 2.0, about 0.5X to about 1.5X, about 1.0X to about 2.5X, about 1.0X to about 2.0X, or about 1.5X to about 2.0X), and about 0% to about 0.15% SDS (e.g. about 0% to about 0.12%, about 0% to about 0.10%, about 0% to about 0.08%, about 0% to about 0.06%, about 0% to about 0.04%, about 0% to about 0.02%, about 0.01% to about 0.10%, about 0.01% to about 0.06%, about 0.02% to about 0.10%, about 0.04% to about 0.10%, about 0.04% to about 0.08%, about 0.04% to about 0.06%, about 0.06% to about 0.10%, about 0.06% to about 0.08%, or about 0.08% to about 0.10%, or 0%, about 0.1%, about 0.2%, about 0.3%, about 0.4%, about 0.5%, about 0.6%, about 0.7%, about 0.8%, about 0.9%, about 0.10%, about 0.11%, about 0.12%, about 0.13%, about 0.14%, or about 0.15%).

In some embodiments, the low stringency buffer includes about 1X SSC and about 0.1% SDS.

In some embodiments of any of the methods described herein, the at least one washing step includes washing using a high stringency buffer (e.g., any of the exemplary high stringency buffers described herein) at a temperature of about 45 °C to about 75 °C (e.g., about 45 °C to about 70 °C, about 45 °C to about 60 °C, about 45 °C to about 50 °C, 50 °C to about 60 °C, 50 °C to about 55 °C, 54 °C to about 60 °C, 54 °C to about 58 °C, about 60 °C to about 65 °C, about 60 °C to about 64 °C, 60 °C to about 62 °C, about 62 °C to about 65 °C, about 62 °C to about 64 °C, about 50 °C, about 52 °C, about 54 °C, about 56 °C, about 58 °C, about 60 °C, about 62 °C, about 64 °C), for about 1 minute to about 10 hours (e.g., about 1 minute to about 6 hours, about 1 minute to about 4 hours, about 1 minute to about 2 hours, about 1 minute to about 1 hour, about 1 minute to about 45 minutes about 1 minute to about 30 minutes, about 1 minute to about 25 minutes, about 1 minute to about 20 minutes, about 1 minute to about 15 minutes, about 1 minute to about 10 minutes, about 1 minute to about 5 minutes, about 5 minutes to about 10 hours, about 5 minutes to about 5 hours, about 5 minutes to about 2 hours, about 5 minutes to about 1 hour, about 5 minutes to about 45 minutes, about 5 minutes to about 30 minutes, about 5 minutes to about 25 minutes, about 5 minutes to about 20 minutes, about 5 minutes to about 15 minutes, about 5 minutes to about 10 minutes, about 15 minutes to about 10 hours, about 15 minutes to about 8 hours, about 15 minutes to about 6 hours, about 15 minutes to about 4 hours, about 15 minutes to about 2 hours, about 15 minutes to about 1 hour, about 15 minutes to about 45 minutes, about 15 minutes to about 30 minutes, about 15 minutes to about 25 minutes, about 15 minutes to about 20 minutes, about 30 minutes to about 10 hours, about 30 minutes to about 4 hours, about 30 minutes to about 2 hours, about 30 minutes to about 1 hour, about 30 minutes to about 45 minutes, about 45 minutes to about 10 hours, about 45 minutes to about 6 hours, about 45 minutes to about 4 hours, about 45 minutes to about 1 hour, about 1 hour to about 10 hours, about 1 hour to about 8 hours, about 1 hour to about 6 hours, about 1 hour to about 5 hours, about 1 hour to about 4 hours, about 1 hour to about 2 hours, about 2 hours to about 10 hours, about 2 hours to about 8 hours, about 2 hours to about 6 hours, , about 2 hours to about 4 hours, about 4 hours to about 10 hours, about 4 hours to about 8 hours, about 4 hours to about 6 hours, about 5 hours to about 10 hours, about 5 hours to about 6 hours, or about 6 hours to about 10 hours).

In some embodiments, the high stringency buffer comprises about 0.1X to about 0.5X SSC (e.g., about 0.10X to about 0.40X, about 0.10X to about 0.30X, about 0.10X to about 0.20X, about 0.15X to about 0.35X, about 0.20X to about 0.5X, about 0.20X to about 0.40X, about 0.20X to about 0.30X, about 0.5X to about 0.25X, about 0.30X to about 0.50X, about 0.30X to about 0.40Xabout 0.40X to about 0.5X about 0.45X to about 0.50X, or about 0.10X, about 0.15X, about 0.20X, about 0.25X, about 0.30X, about 0.35X, about 0.40X, about 0.45X, or about 0.50X SSC), and optionally further includes a detergent (e.g., SDS).

In some embodiments, the high stringency buffer further comprises about 0% to about 0.15% SDS (e.g. about 0% to about 0.14%, about 0% to about 0.12%, about 0% to about 0.10%, about 0% to about 0.08%, about 0% to about 0.06%, about 0% to about 0.04%, about 0% to about 0.02%, about 0.01% to about 0.10%, about 0.01% to about 0.08%, about 0.01% to about 0.06%, about 0.01% to about 0.04%, about 0.01% to about 0.02%, about 0.02% to about 0.10%, about 0.02% to about 0.08%, about 0.02% to about 0.06%, about 0.02% to about 0.04%, about 0.04% to about 0.10%, about 0.04% to about 0.08%, about 0.04% to about 0.06%, about 0.06% to about 0.10%, about 0.06% to about 0.08%, or about 0.08% to about 0.10%, or 0%, 0.1%, 0.2%, 0.3%, 0.4%, 0.5%, 0.6%, 0.7%, 0.8%, 0.9%, 0.10%, 0.11%, 0.12%, 0.13%, 0.14%, or 0.15% SDS).

**Isolating step**

In some embodiments of any of the methods provided herein, the method includes an isolating step. In some embodiments, the hybridization product is isolated using a magnetic bead, chromatography resin (e.g., an agarose bead), membrane, firm, sensor, or a chip that has a covalently attached moiety that binds specifically to a tag covalently linked to the probe (e.g., any of the tags and/or probes described herein). Additional exemplary methods for performing the isolating step are known in the art.

**One or More Additional Steps**

In some embodiments of any of the methods provided herein, one or more additional steps can be performed before and/or after the capturing step (as shown in FIG. 1).

In some embodiments, the one or more (e.g., two, three, four, or five) additional steps performed before the capturing step include: fragmenting DNA (e.g., fragmenting mammalian genomic DNA), performing end repair, dA-tailing, ligating adapters, and/or performing PCR amplification. In some embodiments, the one or more additional steps performed before the capturing step include: fragmenting DNA (e.g., fragmenting mammalian genomic DNA), performing end repair, dA-tailing, ligating adapters, and performing PCR amplification. DNA fragmentation can be performed using a French press, a restriction enzyme digest, DNA shearing (e.g., acoustic shearing, hydrodynamic shearing (e.g., point-sink shearing or needle sheering), nebulization, microwave irradiation, or sonication. See, e.g., Knierim et al. (2011) PLoS One 6(11): e28240; Yang and Hang (2013) J. Biomol. Tech. 24(2): 98-103; and Sapojnikova et al. (2017) J. Biotechnology 256: 1-5. Various commercially available kits can be used to perform end repair and dA-tailing. After DNA fragmentation, end repair is performed in order to generate blunt end 5' and 3' DNA fragments. In some embodiments, end repair is performed using a Klenow DNA polymerase, a T4 DNA polymerase, and/or a T4 polynucleotide kinase (T4 PNK). dA-tailing incorporates one or more non-templated deoxyadenosine monophosphate (dAMP) on the 3' end of a blunt DNA fragment. dA-tailing is performed to prevent concatamer formation. In some embodiments, dA-tailing is performed using a Klenow enzyme that lacks 5' to 3' exonuclease activity and lacks 3' to 5'exonuclease activity. Adaptor ligation includes ligating a short chemically synthesized single- or double-stranded oligonucleotide (i.e., an adaptor) to one or both ends of a nucleic acid. See, e.g., Tsoktouridis et al. (2005) BioTechniques 38(6): 885-888; Aigrain et al. (2016) BMC Genomics 17(1): 1; and Raine et al. (2017) Nucleic Acids Res. 45(6): e36. Polymerase Chain Reaction (PCR) is routinely performed by those skilled in the art. Various methods and kits that can be used to perform PCR are known in the art.

In some embodiments, the one or more additional steps performed after the capturing step include: performing qPCR quantification, washing a sequencing instrument (e.g., a MiSeq®), pooling and denaturing of libraries, and/or running a sequencing instrument (e.g., a MiSeq®). In some embodiments, the one or more additional steps performed after the capturing include: performing qPCR quantification, washing MiSeq®, pooling and denaturing of libraries, and/or running MiSeq®. Relative PCR (qPCR) quantification refers to determining the amount of a target oligonucleotide sequence initially present by monitoring qPCR amplification curves. MiSeq® is an Illumina benchtop sequencing instrument. Various methods and kits for performing qPCR and using a sequencing instrument (e.g., MiSeq®) are known in the art. See, e.g., Bustin et al. (2009) Clin. Chem. 55: 611-622; Karlen et al. (2007) BMC Bioinformatics 8: 131; Ruijter et al. (2013) Methods 59: 32-46; and Quail et al. (2012) BMC Genomics 13: 341.

In some embodiments, the one or more additional steps performed before the capturing step include: fragmenting genomic DNA, performing end repair and dA-tailing, ligating adapters, and/or performing PCR amplification; and the one or more additional steps performed after the capturing step include: performing qPCR quantification, washing a sequencing instrument (e.g., a MiSeq®), pooling and denaturing of libraries, and/or running a sequencing instrument (e.g., a MiSeq®).

In some embodiments, the one or more additional steps performed before the capturing step are fragmenting genomic DNA, performing end repair and dA-tailing, ligating adapters, and performing PCR amplification; and the one or more additional steps performed after the capturing step are performing qPCR quantification, washing a sequencing instrument (e.g., a MiSeq®), pooling and denaturing of libraries, and running a sequencing instrument (e.g., a MiSeq®).

**Tags and Binding Moieties**

In some embodiments of any of the methods described herein, a probe can further includes a tag. In some embodiments, the tag is an internal tag (i.e., the tag is positioned within the entire length of the probe). In some embodiments, the tag is positioned at the 5' end of the probe. In some embodiments, the tag is positioned at the 3' end of the probe.

In some embodiments, the tag is biotin, or a variant thereof. In some embodiments, the tag is streptavidin, or a variant thereof. In some embodiments of any of the methods described herein, the tag and the moiety that specifically binds to the tag can be interchanged. For example, the tag can be biotin, or a derivative thereof, and the moiety that specifically binds to the tag is avidin, or a derivative thereof. In other examples, the tag can be avidin, or a derivative thereof, and the moiety that specifically binds to the tag is biotin. Additional examples of tags and corresponding binding moieties are known in the art.

The tags and the moieties that specifically bind to the tag provided herein can bind with a dissociation equilibrium constant ($K_D$) of less than $1 \times 10^{-7}$M, less than $1 \times 10^{-8}$M, less than $1 \times 10^{-9}$M, less than $1 \times 10^{-10}$M, less than $1 \times 10^{-11}$ M, less than $1 \times 10^{-12}$M, less than $1 \times 10^{-13}$ M, less than $1 \times 10^{-14}$ M. less than $1 \times 10^{-15}$ M, or less than $1 \times 10^{-16}$ M. In some embodiments, the tags and the moieties that bind to the tag provided herein can bind with a $K_D$ of about $1 \times 10^{-4}$ M to about $1 \times 10^{-6}$ M, about $1 \times 10^{-5}$ M to about $1 \times 10^{-7}$ M, about $1 \times 10^{-6}$ M to about $1 \times 10^{-8}$ M, about $1 \times 10^{-7}$ M to about $1 \times 10^{-9}$ M, about $1 \times 10^{-8}$ M to about $1 \times 10^{-10}$ M, about $1 \times 10^{-9}$ M to about $1 \times 10^{-11}$ M, about $1 \times 10^{-9}$ M to about $1 \times 10^{-12}$ M, about $1 \times 10^{-9}$ M to about $1 \times 10^{-13}$ M, about $1 \times 10^{-9}$ M to about $1 \times 10^{-14}$ M, about $1 \times 10^{-9}$ M to about $1 \times 10^{-15}$ M, about $1 \times 10^{-10}$ M to about $1 \times 10^{-15}$ M, about $1 \times 10^{-10}$ M to about $1 \times 10^{-14}$ M, about $1 \times 10^{-10}$ M to about $1 \times 10^{-13}$ M, about $1 \times 10^{-13}$ M to about $1 \times 10^{-15}$ M, or about $1 \times 10^{-14}$ M to about $1 \times 10^{-15}$ M. In some embodiments, the tags and the moieties that specifically bind to the tag provided herein can bind with a $K_D$ of about 1.1 nM to about 500 nM, or about 2.0 nM to about 6.7 nM.

In some embodiments of any of the methods described herein, the moiety that binds to the tag is covalently attached to a bead (e.g., a magnetic bead, a chromatography resin (e.g., an agarose bead), or a polymer bead), a particle (e.g., a microparticle or a nanoparticle). In some embodiments of any of the methods described herein, the moiety that specifically binds to the tag is covalently attached to a bead. In some embodiments, the bead is a magnetic bead.

**Compositions**

Also provided herein are compositions comprising or consisting of a liquid, where the liquid includes: about 0.5 M to about 8.0 M (or any of the subranges of this range described herein) TMAC; about 10 mM to about 200 mM (or any of the subranges of this range described herein) Tris (pH 8.0); about 1X to about 5X (or any of the subranges of this range described herein) Denhardt's solution; about 0.01% to about 0.2% (or any of the subranges of this range described herein) Tween-20; about 0.5 mM to about 15 mM (or any of the subranges of this range described herein) EDTA; and about 0.5% to about 25% (or any of the subranges of this range described herein) formamide (v/v).

In some embodiments of these compositions, the liquid includes about 2.7 M TMAC, about 50 mM Tris (pH 8.0), about 2.5X Denhardt's Solution, about 0.010% Tween-20, about 6 mM EDTA, and about 20% formamide.

In some embodiments of any of the compositions described herein, the composition consists of the liquid.

The compositions may be advantageously used in any of the aforementioned hybridization/capture methods.

**Kits**

Also provided herein are kits containing one or more (e.g., at least 2, 3, 4, 5, 6, 7, 8, 9, 10, 12, 14, 16, 18, 20) of any of the probes and buffers (e.g., one, two, or three of a hybridization buffer, low stringency buffer, and high stringency buffer described herein). Also provided herein are kits that include any of the compositions described herein. In some embodiments, the kits can include instructions for performing any of the methods described herein.

In some embodiments, the kit can contain at least two primers (e.g., at least 4, 6, 8, 10, 12, 14, 16, 18, 20, 22, 24, 26, 28, 30, 32, 34, 36, 38, 40, 42, 44, 46, 48, or 50) for amplifying a target oligonucleotide sequence (e.g., a target oligonucleotide sequence within a library of nucleic acids (e.g., any of the libraries described herein)). In some embodiments, the kit can contain two or more sets of primers, wherein one set includes a forward primer and a reverse primer (e.g., 3, 4, 5, 6, 7, 8, 9, 10, 11, 12, 13, 14, 15, 16, 17, 18, 19, 20, 21, 22, 23, 24, 25, 26, 27, 28, 29, 30,31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120,121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 178, 179, 180, 181, 182, 183, 184, 185, 186, 187, 188, 189, 190, 191, 192, 193, 194, 195, 196, 197, 198, 199, or 200 pairs of primers) for amplifying a target oligonucleotide sequence (e.g., a target oligonucleotide sequence within a library of nucleic acids (e.g., any of the libraries described herein)). In some embodiments, the kits can contain a set of multiplex primers, wherein one set includes a forward multiplex primer and a reverse multiplex primer.

In some embodiments, the kit can contain a probe that includes a sequence that is complementary to the target oligonucleotide sequence and includes an internal tag, or a tag at its 5' or 3' end. In some embodiments, the tag is a fluorophore, a radioisotope, an enzyme, biotin, or streptavidin. In some embodiments, the tag is streptavidin, or a variant thereof. In some embodiments, the kit can further include a magnetic bead, chromatography resin (e.g., an agarose bead), membrane, firm, sensor, or a chip that has a covalently attached moiety that binds specifically to a tag covalently linked to the probe (e.g., any of the tags and/or probes described herein).

The kit may be advantageously used in any of the aforementioned hybridization/capture methods.

**EXAMPLES**

The invention is further described in the following examples, which do not limit the scope of the invention described in the claims.

**Example 1 - Assay Method**

The assay provided herein converts genomic DNA into Illumina-sequencing-ready libraries and sequences the libraries in gene regions known to house variants with large effects on cancer risk. Mendelian, highly penetrant variants for cancer risk are reported to the patient. As the landscape of genes that are important to hereditary cancer risk grows, it is becoming increasingly important to develop methods to interrogate multiple genes that may carry mutations. Individuals with a family history of breast cancer, for instance, may house mutations in genes other than BRCA1 and BRCA2. In order to provide a more comprehensive view of a patient's risk, sequencing of genomic DNA with multi-gene panels has become common [2, 3]. Testing hereditary breast and/or ovarian cancer (HBOC) with a multi-gene panel, for instance, may identify 40% more individuals with hereditary cancer gene mutations than testing BRCA1 and BRCA2 alone [4]. The multi-gene panel approach also eases the testing burden by reducing follow-up testing after negative results (from a smaller testing region), and by simplifying the constellation of tests offered.

The gene panel (Table 1) was designed to allow analysis of genes and genetic variants associated with predisposition to cancer. It targets germline variants detectable in white blood cell DNA. The panel was selected based on systematic, expert review of the scientific literature, to identify genes that predispose a subject to cancer. It focuses on genes for which there is already strong evidence of an association with cancers. It does not contain the many genes/variants for which a putative role in cancer has been proposed, but not proven.

**Table 1. Genes included in the panel.**

| AIP | ALK | APC | ATM | BAP1 | BLM | BMPR1A | BRCA1 | BRCA2 | BRIP1 |
|-----|-----|-----|-----|------|-----|--------|-------|-------|-------|
| BUB1B | CDC73 | CDH1 | CDK4 | CDKN1C | CDKN2A | CEBPA | CEP57 | CHEK2 | CYLD |
| DOB2 | DICER1 | DIS3L2 | EGFR | EPCAM | ERCC2 | ERCC3 | ERCC4 | ERCC5 | EXT1 |
| EXT2 | EZH2 | FANCA | FANCB | FANCC | FANCD2 | FANCE | FANCF | FANCG | FANCI |
| FANCL | FANCM | FH | FLCN | GATA2 | GPC3 | HNF1A | HOXB13 | HRAS | KIT |
| MAX | MEN1 | MET | MITF | MLH1 | MSH2 | MSH6 | MUTYH | NBN | NF1 |
| NF2 | NSD1 | PALB2 | PHOX2B | PMS1 | PMS2 | PPM1D | PRF1 | PRKAR1A | PTCH1 |
| PTEN | RAD51C | RAD51D | RB1 | RECQL4 | RET | RHBDF2 | RUNX1 | SBDS | SDHAF2 |
| SDHB | SDHC | SDHD | SLX4 | SMAD4 | SMARCB1 | STK11 | SUFU | TMEM127 | TP53 |
| TSC1 | TSC2 | VHL | WT1 | WRN | XPA | XPC | | | |

Sample preparation converts genomic DNA into sequence-ready library, and enriches that library for exons in genes of interest using DNA-RNA hybrid selection [5] (FIG. 1). High coverage sequence data is generated with Illumina's MiSeq® sequencer, and variants are called with an automatic analysis pipeline. The validation of the process utilizes end-to-end processing of well-known samples to characterize the sensitivity, specificity, accuracy, precision, and other metrics for the entire assay workflow.

CEPH Pedigree 1463 contains 17 samples over three generations. The pedigree is part of the 1000G and HapMap projects (www.1000genomes.org/about; hapmap.ncbi.nlm.nih.gov) and NA12878 is often used as a reference sample, or internal control by laboratories. Platinum Genome Project sequenced all 17 samples and generated high-quality consensus calls from the Genome In A Bottle Consortium (which provides data for NA12878).

The assay was validated using 19 unique "Golden Standard" reference samples run over 38 different library preparations. Analytical sensitivity, specificity, and accuracy is assessed over 19 unique samples - each of which is expected to have around 100 single nucleotide variants. After processing from DNA input, through variant calling, the data produced is compared to the high-quality reference calls to assess assay performance. Two of the samples, were run after being freeze-thawed 10X in order to measure the interference from improper sample handling. In addition, six of the samples from the sensitivity/specificity/accuracy test were rerun to measure precision.

The assay utilizes genomic DNA (gDNA) extracted from blood as its input. CellMax Life requires that gDNA sent to the lab for processing is extracted with the use of a column kit similar to Life Technologies' PureLink Genomic DNA kit. gDNA is abundant in blood, and DNA extracted with the use of commercial kits (as opposed to home-brew salting out procedures) produces DNA free from contaminants that effect long term storage.

*Sequencing Library Preparation from Genomic DNA*

In order to sequence genomic DNA with Illumina's MiSeq®, genomic DNA - which starts out as fragments over 10kb - must be fragmented (FIG. 1). The enzyme Fragmentase (commercially available from NEB) was used to fragment DNA. This method requires less sample handling than other methods such as acoustic shearing with Covaris. During the incubation, DNA is randomly cleaved (double-stranded cut) by Fragmentase. The incubation period and enzyme:DNA copy ratio are both major determinants of the final size distribution of the fragmented DNA. Incubating for too long, for instance, will result in over-fragmented DNA. To avoid major variations in the size distributions of fragmented DNA, the cleavage reaction is promptly stopped with ethylenediaminetetraacetic acid (EDTA), and the input DNA amount is set to a standard 1.5μg for all samples. Minor variations are to be expected due to technician handling differences, and are mitigated by the inclusion of multiple size selection steps.

Once fragmented, the DNA is prepared for adapter ligation (FIG. 2). The DNA fragments contain 5' and 3' overhangs that are blunt-ended by T4 & Taq DNA Polymerase, and the blunt-ended DNA is 3' phosphorylated by T4 PNK (all enzymes are from NEB). These three steps are performed in the same tube. The tube is then heated from 20 °C to 65 °C, where an A-tail is added. NEB's Quick Ligase, along with buffer and adapters, are added directly to the End-Repair/dA-tailing mix. The use of an enzyme cocktail for end-repair and dA-Tailing, the addition of ligation reagents directly to the previous reaction, and the lack of clean-up steps reduces sample loss and potential contamination due to operator handling.

Adapter-ligated DNA is cleaned then PCR amplified with KAPA's Library Amplification Kit (FIG. 2). KAPA's polymerase was chosen due to its reported high fidelity and low GC bias. The cleaned, amplified libraries' concentration is measured with a Qubit BR kit and the size distribution is recorded with an Agilent BioAnalyzer 1000 chip (FIG. 1).

*Library Enrichment for Targeted Regions*

Amplified libraries could be sequenced directly after PCR amplification, but doing so would result in sequencing the entire human genome (~3 Gigabases). Most of the data would be unusable for cancer risk assessment, and sequencing to a great enough depth to call variants would be prohibitively expensive. Instead, the libraries provided herein are enriched for exons in genes with a known relationship to hereditary cancer syndromes using the RNA-DNA hybrid selection method from Gnirke, et al. (Gnirke, Andreas, et al. "Solution hybrid selection with ultra-long oligonucleotides for massively parallel targeted sequencing." Nature Biotechnology 27.2 (2009):182-189).

Probes sequences are designed by tiling exons in targeted genes. Each probe is of fixed length, and probe overlap is increased in regions where sequencing coverage is expected to be low due to GC bias (FIGs. 5-8). After design, all probe sequences have common ends appended. The probe-common-end sequences are sent to a commercial provider for massively parallel DNA oligonucleotide synthesis (AZCO Biotech). The synthesized oligos are subsequently PCR amplified to introduce a T7 promoter. In vitro transcription is performed with the addition of biotin UTP overnight. RNA products are cleaned of DNA with Turbo DNAse and the reaction is purified with a column.

Amplified libraries are pooled and transcribed baits are added for hybridization (using TMAC-based hybridization buffer, described below, and performed at a temperature of about 60 °C to about 70 °C), bead pull-down, and washing (low stringency wash buffer and/or high stringency wash buffer, described below) (FIG. 3). The hybridization takes place for 16 hours, after which RNA baits (and their hybridized DNA libraries) are bound with streptavidin beads (FIGs. 9-10). The beads then go through a mechanical wash with a high salt buffer at room temperature, then multiple high stringency washes with a low salt buffer at high temperature to reduce the number of off-target library molecules that are co-enriched. After enrichment, a second PCR is performed to amplify the enriched libraries.

*MiSeq® Sequencing*

After enrichment, libraries are ready for sequencing with Illumina's MiSeq®. qPCR quantification is used due to its high accuracy, and the results are size-corrected with a library size estimate obtained from the pre-enrichment BioAnalyzer run (FIG. 4).

A stringent bleach wash of the MiSeq® is performed prior to the run to prevent run-to-run read carryover, and the libraries are sequenced with 200 cycle paired-end reads according to Illumina's MiSeq® User Guide. A MiSeq® run control is included in each sequence run to ensure that run quality is sufficient for processing. The run control, PhiX, is provided by Illumina as a prepared library from PhiX DNA. The genomic sequence of PhiX is hard-coded into the MiSeq® run analysis software, and after 25 cycles, any reads originating from PhiX library molecules are recognized and tagged. The use of a well-known sequence allows the MiSeq® software to calculate the actual average error rate of the sequencing run. This serves as a control for sequence quality. In processing PhiX is spiked-in at 1% as recommended by Illumina (Using a PhiX Control for HiSeq Sequencing Runs, downloaded on 11 June 2016).The bioinformatics pipeline is an end-to-end, automated pipeline that starts with raw MiSeq® data, and produces variant calls that have been annotated for expected effect of the variant on protein function. During the processing, multiple QC metrics are enforced to ensure that samples have not been contaminated, and that the data is of sufficient quality to confidently call variants.

Raw MiSeq® data (basecall files) are demultiplexed in order to separate reads originating from different libraries that were pooled in the MiSeq® run. The resultant Fastq files (files with the generated sequence for a library molecule, and the associated quality scores) are filtered to remove content that is of low quality or is from the library's adapter. The filtered Fastq files are aligned to the reference genome (hg19), filtered again for quality and realigned to reduce false positives from insertions or deletions. Variants are called with Varscan and annotated for, and filtered by technical parameters with the GATK based on the GATK's best practice recommendations (2.3-9, The GATK Guide Book Version).

After filtering for technical false positives, the tertiary analysis portion of the pipeline annotates variants for frequency in the general population, computational likelihood that a variant has a pronounced effect on the protein's function, and any assertions made by other clinical laboratories for cancer syndromes in the ClinVar database.

*Assay Quality Control*

The end-to-end process for patient samples is monitored with the inclusion of two batch controls in the processing of each batch: one positive control with a known pathogenic variant, and one negative control from this analytical validation study. The positive control will be processed, and if the known pathogenic variant is not called in the sample, then the batch will be failed. The negative control will be scored similarly. All called variants will be compared against known variants (as in the validation report), if the sensitivity or specificity falls below 99.00% or 99.99% respectively, the batch is failed.

Poor processing, or reagent degradation is monitored at each step, but may also be identified in subsequent steps. If enzymatic fragmentation of genomic DNA results in over or under fragmented DNA, the size selection step should enrich for the correct size (which would be a minority) and the yield QC should fail.

The MiSeq® and sequence data QC metrics monitor for sufficient data to call variants. While a number of steps go into sequencing the samples, only the final coverage for regions in the reportable range affects the final results. If any of the individual steps is not optimal, the final coverage is affected. For example, if the quality of the raw reads is low due to overclustering, after the pipeline filters the data, and only high quality data remains, the samples should fail the coverage metric.

By using phiX as a reference standard for sequencing quality, the authors did not rely on self-reporting by the MiSeq® for errors: if an insidious error occurs, and the MiSeq® does not report lower quality scores, the PhiX error rate metric will be triggered.

**Example 2 - Design Efficiency**

The targeted genomic regions for which variants can be called with high confidence. It is important to ensure that variant calling is only performed in regions, which will produce high quality data, which consistently pass QC. To measure the amount of the target set that is retained, the design efficiency metric was defined.

$$\textit{Design Efficiency} = \textit{(base pairs in reportable range / base pairs targeted) X 100}$$

Exon regions for 98 genes, and regions reported as high confidence by the GIAB consortium for NA12878. The intersection of the GIAB high confidence regions (for the whole genome) with target regions was defined to be the reportable range. In order to define the target regions, exons regions were selected from 98 genes with known relationships to hereditary cancer syndromes. The target regions, are then intersected with the GIAB high-confidence regions to produce the reportable range.

The reportable range of the assay includes exons for 98 genes (Table 1) across a 200,000 base pair target region. The reportable range is 82% of the target regions (design efficiency of 82%). As the target regions are almost exclusively the entire exon regions for the gene list, the reportable range is 82% of the exon regions for the gene list. Design efficiency is 82% of the targeted regions.

One approach to define the regions of the target set that provide high confidence variant calls would be to enumerate the types of problematic regions that cause false positives and false negatives. Examples of problematic regions and their effect could include repeat regions (which cause mapping, and insertion/deletion issues) and high GC regions (which can cause low coverage in hybridization enrichments). Once the types of regions are enumerated, then a metric for "good" and "bad" can be defined and a cutoff can be trained. Training the cutoff is often largely based on heuristic analysis, however, as reference data in poor quality regions is hard to obtain.

By contrast to manually defining possible error profiles, and omitting regions which likely fit into the "bad" end of the spectrum, reportable regions were defined as being the regions where high-quality data is reported by the GIAB consortium for NA12878. The region houses a repeat sequence that would likely cause false positives. This illustrates the general assumption that if the multiple, high quality, data sets that comprise the GIAB dataset for a region cannot create consensus calls, high quality variant calling using one technology is likely not feasible in this region. Of note using the Platinum Genomes data as a basis for an assay's reference range would not be possible as not as many different sequencing technologies were utilized, and some high-quality variants were called with only one pipeline with aid of pedigree constraints.

## Example 3 - Analytical Sensitivity

Analytical sensitivity is defined as the likelihood that a variant will be called if present in the source material [18].

$$Analytical\ Sensitivity = (variants\ correctly\ called\ by\ processing/\ variants\ expected\ from$$

$$reference\ data)\ X\ 100$$

Variant calls were produced by the automated pipeline for each sample, but were not annotated for clinical metrics. Reference SNV calls were pulled from the sources listed and filtered so that variant rows with loci in the reportable range were retained. To calculate the sensitivity, variants expected from the reference data were compared to the call set. Variants from 18 samples were called with 100% of the expected variants identified correctly, and one variant was missed in sample NA24143. The measured sensitivity for all 19 samples in the analytical validation is calculated below:

$$Analytical\ Sensitivity = (1841\ correctly\ called\ variants/\ 1842\ expected\ variants)\ X\ 100 = 99.95\%.$$

The high sensitivity of the variant calls is enabled by high quality data as opposed to complex variant calling techniques. Raw data (data not shown) shows a clear separation between expected variant positions and false positives: the false positives have significantly lower allele frequencies. Through the use of strict quality filters and high sequencing coverage, individual errors are drowned out by high quality reference alleles.

## Example 4 - Analytical Specificity

The probability that the assy will not detect a sequence variation when none are present [18].

$$Analytical\ Specificity = (reference\ alleles\ correctly\ called/\ reference\ alleles\ expected$$

$$from\ the\ reference\ data)\ X\ 100$$

Eight samples were processed. Variant calls were produced by the automated pipeline described herein for each sample, but were not annotated for clinical metrics. Reference SNV calls were pulled from the sources and filtered so that variant rows with loci in the reportable range were retained. To calculate the specificity, variants called by processing were compared to the reference call set. Any variants called, but not expected, would be marked as a false positive. Variants from 19 samples were called with no false positives. The measured specificity for all 19 samples in the analytical validation is calculated below:

$$Analytical\ Specificity = (3898611\ correct\ reference\ calls\ /\ 3898611\ expected\ reference\ calls)\ X\ 100 = 100\%.$$

**Example 5 - Accuracy**

The degree of agreement between the nucleic acid sequences derived form the assay and a reference sequence [18].

$$Accuracy = (number\ of\ correct\ variant\ calls/\ total\ number\ of\ variant\ calls)\ X\ 100$$

Variant calls were produced by the automated pipeline for each sample, but were not annotated for clinical metrics. Reference SNV calls were pulled from the sources and filtered so that variant rows with loci in the reportable range were retained. To calculate the accuracy, variants called by processing were compared to the reference call set. Any variants called, but not expected, would be marked as a false positive, and any calls missed, but that were expected, were marked as a false negative. The measured accuracy was over 99.99%. Only one incorrect call was made (a false negative in sample NA24143).

$$Accuracy = ((3898611\ correct\ reference + 1841\ correct\ variant)\ /\ 3900453\ total\ variant\ \&\ reference\ calls)\ X\ 100 = > 99.99\%$$

**Example 6 - Precision**

Closeness of agreement between independent test results obtained under stipulated conditions [18]. Precision of the assay is determined by measuring reproducibility.

$$Reproducibility = (number\ of\ calls\ in\ agreement\ across\ conditions\ for\ a\ sample/\ total\ number\ of\ calls\ in\ across\ conditions\ for\ a\ sample)\ X\ 100$$

Eleven samples from the analytical validation run were run again as replicates. Variant calls were be produced by the automated pipeline for each replicate. SNV calls in the reportable range were compared. In order for a variant to be categorized as in agreement between the replicates, the variant call was required to be at the same loci, and call the same alternate allele with the same genotype. (For example, for a reference homozygous position of "A" that is converted to a heterozygous "C" call, both samples should call both the variant "C" and classify the variant as heterozygous.)
Reproducibility for all 6 replicated samples (processed by different technicians, on different weeks) was 100%:

$$Reproducibility = (2258157\ calls\ in\ agreement\ /\ 2258157\ total\ calls)\ X\ 100 = 100\%$$

The calculation above shows that the reproducibility for eleven samples, each with 205, 287 loci.
To better show the agreement, or disagreement, between variant calls across runs, the zygosity for all variant positions for each replicate was plotted (FIG. 11). One representative sample is shown below. Any disagreeing calls would be seen as the box (run 1 marker) and X (run 2 marker) not aligning on the Y-axis. Note that, for simplicity, the chart does not show alternate allele (A, C, G, or T call) information, but the alternate alleles were compared across call sets.

**Example 7 -Interference**

The effect of interfering substances or conditions on the input to the assay (gDNA). The interfering effect of simulated poor handling of gDNA was determined by measuring reproducibility after freeze-thawing the samples 10 times. Extended shipment times were tested by shipping samples to Taiwan, then shipping the same samples back to the US again. Two samples from the analytical validation run were run after repeated freeze-thawing to simulate samples that have to been rerun due to quality control (QC) failure. Six samples from the analytical validation run were used for the shipment test to ensure that DNA shipped from Taiwan can be utilized in the assay provided herein. Variants were called and compared as in the precision test in Example 7. Reproducibility for both samples after freeze-thaw was 100%.

$$\text{Reproducibility (Freeze-Thaw)} = (410574 \text{ calls in agreement} / 410574 \text{ total calls}) \times 100$$

$$= 100\%$$

$$\text{Reproducibility (Shipment)} = (1231722 \text{ calls in agreement} / 1231722 \text{ total calls}) \times 100 =$$

$$100\%$$

The calculation above shows the reproducibility for both the freeze-thaw and shipment tests. Of note, each sample is interrogated at 205,287 loci.

**Example 8 - Average Sequencing Coverage**

Sequencing to too great depth leads to increased cost to the consumer, but having too few reads often results in false negatives, as many regions are below coverage thresholds for variant identification. The average target coverage is defined here as the average number of reads that contribute to a base call at a given position across all positions in the target set. Average target coverage is a useful metric as it can be easily monitored (along with coverage evenness) for QC in routine processing. Despite the fact that heterozygous SNVs can be called with 10 reads for the variant allele, the average target coverage must be much higher to ensure that > 99% of targeted positions can be called. This high average coverage requirement is due variability in both the measured allele frequency, and the base coverage depth across targeted regions.

Downsampling analysis across six samples shows that in order to not artificially limit sensitivity, samples must have an average target coverage of over 100X (FIG. 12). With six samples (and 570 variant calls) a large swath of genomic variants are queried for variant calls (and by proxy: sufficient coverage for variant calls).

**Example 9 - Protocols**

*Dry Down DNA For Capture*

1. Pool libraries for capture
2. Prepare DNA for 3 pools
a. Prepare DNA, blocking DNA and oligos MM and aliquot to strip PCR tubes

| Reagent | 1x | 3.0X |
| --- | --- | --- |
| Cot-1 DNA (1mg/ml) | 5.0ul | 15.0ul |
| Salmon Sperm DNA (10mg/ml) | 2.5ul | 7.5ul |
| xGen TruSeq LT-p7 (6nt) (1mM) | 1.0ul | 3.0ul |
| xGen HT-i5 (1mM) | 1.0ul | 3.0ul |
| Total | 9.5ul | 28.5ul |

3. Dry down libraries for capture

    a. Add blocking oligos to pooled libraries

    b. Split each pooled capture into two PCR strip tubes

    c. Dry down in PCR tubes in PCR machine

        i. PCR machine at 45 °C (w/out heated lid)

        ii. Dry down to ~2ul per tube (4$\mu$l per capture pool)

        iii. If final volume for pooled capture is <4$\mu$l, add $H_2O$ to bring volume up

*Prepare In-House Buffers*

1. Prepare TMAC-based hybridization buffer
a. Add 300µl of 10% Tween-20 with 700µl of H2O to final 3%

| Reagent | Stock | 2.00X | In-Hyb. (when diluted) | 190ul | 1000ul |
|---|---|---|---|---|---|
| Tris, pH8.0 | 5.00M | 100mM | 50mM | 3.8ul | 20.0ul |
| 100X Denhardt's Solution | 100X | 5.0X | 2.5X | 9.5ul | 50.0ul |
| H2O | | | | 0ul | -0.7ul |
| Tween-20 | 3% | 0.020% | 0.010% | 1.3ul | 6.7ul |
| .5M EDTA | 0.5M | 12mM | 6mM | 4.6ul | 24.0ul |
| TMAC | 6M | 5.4M | 2.7M | 171ul | 900.0ul |
| Total | | | | 190ul | 1000ul   at 2X |

2. Add formamide to hybridization buffer to final 1.43X (hybrid-formamide buffer).

| Condition | Hyb Buff. | Formamide | Total |
|---|---|---|---|
| 20% Formamide | 200.0ul | 80.0ul of 100% | 280.0ul |

3. Prepare Bead Wash Buffer, 1M NaCl

| Reagent | 25ml |
|---|---|
| H2O | 19.7ml |
| 5M NaCl | 5.0ml |
| 1M Tris-Cl pH 8.0 | 250ul |
| .5M EDTA | 50ul |

4. Prepare Low Stringency Wash Buffer

| Reagent | 25ml |
|---|---|
| H2O | 23.5ml |
| 20X SSC | 1.25ml |
| 10% SDS | 250ul |

5. Prepare High Stringency Wash Buffer (0.25X SSC)

| Reagent | 25.0mL | 50.0mL |
|---|---|---|
| H2O | 24.7ml | 49.4ml |
| 20X SSC | 312.5ul | 625.0ul |

*Hybrid Selection*

1. Hybrid recipe:

| Reagent | 1x |
|---|---|
| Hyb. Buff. + Form. (1.43X) | 12.0ul |
| Pool. Lib. + BLK Oligos | 4.0ul |
| Pool A/B Probes (In hyb. buff.) | 4.0ul |
| Total | 20.0ul |

2. Resuspend blocking DNA and oligos in hybridization buffer + formamide

a. Add 6 μl of 1.43X hybrid-formamide buffer to 4μl of dried down DNA,

b. Rinse tube, transfer to another tube, rinse tube, transfer ~16μl to PCR plate

3. Dilute IDT probes to 1X
a. Dilute probes to 4X in 1.43X hybrid buffer + formamide.

a. Add 2μl of 10X Pool A to 18μl of 1.43 hybrid buffer + formamide.

b. Add 1μl of 10X Pool B to 9μl of 1.43 hybrid buffer + formamide.

b. Combine 1X Pools A & B

| Probes | 1X Baits | 2X Baits |
|--------|----------|----------|
| Pool A | 3.8ul | 7.6ul |
| Pool B | 1.2ul | 2.4ul |
| Total | 5.0ul | 10.0ul |

4. Denature DNA

c. Put DNA + BLK oligos in hybrid buffer + formamide into polymerase chain reaction (PCR) machine (use PCR plate & strip cap)

d. Start PCR program:

i. Denature at 95°C for 10 minutes
ii. Cool down to 65°C, open cap and add 4μl of 1X probes in buffer with multi-channel pipet (set volume at 5μl)
iii. Pipet 10 times
iv. Use strip caps & film cover

e. Incubate at 65°C for 16h

i. Use heated lid

*Bead Binding & Wash*

1. Prepare Dynabeads™ MyOne™ Streptavidin T1 beads

a. For one capture (scale up as necessary):

i. Add 50μl of beads to 165μl of Bead Wash Buffer, vortex briefly
ii. Wash 3X with 165μl of Bead Wash Buffer, quick vortex after adding buffer
iii. Re-suspend in 165μ of Bead Wash Buffer

2. Bind hybrid products to T1 beads

a. Use P-200 to pipet 30μl of beads
b. Dispense bead to hybrid reaction
c. Transfer reaction from PCR machine to the rest of beads

i. Reaction transferred 45 - 50μl, pipet few times quickly
ii. Vortex for 5seconds
iii. Incubate at room temperature for 30mininutes
iv. Invert occasionally (1X at 15 min or 2X every 10 min)

d. Wash with Low Stringency Buffer (with sodium dodecyl sulfate (SDS))

   i. Re-suspend beads in 165$\mu$l of LSB and incubate for 15min at room temperature

e. Wash with Foundation High Stringency Buffer (HSB) (0.25X SSC, without SDS)

   i. Re-suspend beads in 165$\mu$l of pre-warmed HSB (55°C)
   ii. Vortex and incubate at 55°C for 10min in the heat block (set 55°C)
   iii. Repeat for a total of 4 washes
   iv. Re-suspend beads in 40$\mu$l of H$_2$O

[1] "Couch, Fergus J., and Barbara L. Weber. "Mutations and Polymorphisms in the familial early onset breast cancer (BRCA1) gene." Human mutation 8.1 (1996): 8-18.".

[2] "Chong, Hansook Kim, et al. "The validation and clinical implementation of BRCAplus: a comprehensive high-risk breast cancer diagnostic assay." PLoS One 9.5 (2014): e97408.".

[3] "Lincoln, Stephen E., et al. "A Systematic comparison of traditional and multigene panel testing for hereditary breast and ovarian cancer genes in more than 1000 patients." The Journal of Molecular Diagnostics 17.5 (2015): 533-544.".

[4] "Desmond, Andrea, et al. "Clinical actionability of multigene panel testing for hereditary breast and ovarian cancer risk assessment." JAMA oncology 1.7 (2015): 943-951.".

[5] "Gnirke, Andreas, et al. "Solution hybrid selection with ultra-long oligonucleotides for massively parallel targeted sequencing." Nature biotechnology 27.2 (2009): 182-189.".

[6] www.1000genomes.org/about.

[7] hapmap.ncbi.nlm.nih.gov

[8] "IlluminaPlatinumGenomes-user-guide.pdf".

[9] www.personalgenomes.org

[10] "Zook, Justin M., et al. "Extensive sequencing of seven human genomes to characterize benchmark reference materials." bioRxiv (2015): 026468.".

[11] "Eberle, Michael A., et al. "A reference dataset of 5.4 million human variants validated by genetic inheritance from sequencing a three-generation 17-member pedigree." bioRxiv (2016): 055541.".

[12] "Zook, Justin M., et al. "Integrating human sequence data sets provides a resource of benchmark SNP and indel genotype calls." (2014).".

[13] www.horizondiscovery.com/reference-standards/q-seq-hdx/genome-in-a-bottle (pulled 10th June 2016).

[14] "Bronner, Iraad F., et al. "Improved protocols for illumina sequencing." Current Protocols in Human Genetics (2014): 18-2.".

[15] research.fhcrc.org/content/dam/stripe/hahn/methods/mol_biol/SPRIselect%20User%20Guide.pdf.

[16] "Using a PhiX Control for HiSeq Sequencing Runs, downloaded on 11 June 2016".

[17] "2.3-9, The GATK Guide Book Version".

[18] "Gargis, Amy S., et al. "Assuring the quality of next-generation sequencing in clinical laboratory practice." Nature biotechnology 30.11 (2012): 1033-1036.".

[19] "Rehm, Heidi L., et al. "ACMG clinical laboratory standards for next-generation sequencing." Genetics in Medicine 15.9 (2013): 733-747.".

[20] "Richards, Sue, et al. "Standards and guidelines for the interpretation of sequence variants: a joint consensus recommendation of the American College of Medical Genetics and Genomics and the Association for Molecular Pathology." Genetics in Medicine (2".

**OTHER EMBODIMENTS**

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

**Claims**

1. A composition comprising or consisting of a liquid, wherein said liquid comprises:

   about 0.5 M to about 8.0 M TMAC;
   about 10 mM to about 200 mM Tris (pH 8.0);
   about 1X to about 5X Denhardt's solution;
   about 0.01% to about 0.2% Tween-20;
   about 0.5 mM to about 15 mM EDTA; and
   about 0.5% to about 25% formamide (v/v).

2. The composition according to claim 1, wherein said liquid comprises about 2.0 M to about 6.0 M TMAC.

3. The composition according to claim 1, wherein said liquid comprises about 5.0 M to about 6.0 M TMAC.

4. The composition according to claim 1, wherein said liquid comprises about 5.4 M TMAC.

5. The composition according to any one of claims 1-4, wherein said liquid comprises about 40 mM to about 60 mM Tris (pH 8.0).

6. The composition according to any one of claims 1-4, wherein said liquid comprises about 100 mM Tris (pH 8.0).

7. The composition according to any one of claims 1-6, wherein said liquid comprises about 2X to about 3X Denhardt's Solution.

8. The composition according to any one of claims 1-6, wherein said liquid comprises about 5X Denhardt's Solution.

9. The composition according to any one of claims 1-8, wherein said liquid comprises about 0.01% to about 0.05% Tween-20.

10. The composition according to any one of claims 1-9, wherein said liquid comprises about 5 mM to about 15 mM EDTA.

11. The composition according to any one of claims 1-10, wherein said liquid comprises about 10% to about 25% formamide (v/v).

12. The composition according to any one of claims 1-10, wherein said liquid comprises about 20% formamide (v/v).

13. The composition according to claim 1, wherein said liquid comprises about 2.7 M TMAC, about 50 mM Tris (pH 8.0), about 2.5X Denhardt's Solution, about 0.010% Tween-20, about 6 mM EDTA, and about 20% formamide.

14. A kit comprising the composition according to anyone of claims 1-13.

15. Use of the composition according to anyone of claims 1-13 or the kit according to claim 14 for performing hybridization/capture methods.

```
┌─────────────────────────┐      ┌─────────────────────────┐      ┌─────────────────────────┐
│     Fragmentation       │      │     Hybridization       │      │   qPCR Quantification   │
│ Incubation And Setup:   │      │    Setup: 1 hour        │      │ Incubation & Setup:     │
│        1 hour           │      │  Incubation: 16 hours   │      │        2 hours          │
│    Ampure: 1 hour       │      └─────────────────────────┘      └─────────────────────────┘
│   Qubit: 30 minutes     │
└─────────────────────────┘
```

**Fragmentation**
*Incubation And Setup: 1 hour*
*Ampure: 1 hour*
*Qubit: 30 minutes*

↓

**End Repair & dA-Tailing**
*Incubation And Setup: 1 hour*

↓

**Adapter Litigation**
*Incubation And Setup: 45 minutes*
*Ampure: 45 minutes*

↓

**PCR Amplification**
*Incubation And Setup: 1 hour*
*Ampure: 1 hour*
*BioAnalyzer: 1 hour; Qubit: 30 min*

**Hybridization**
*Setup: 1 hour*
*Incubation: 16 hours*

↓

**Bead Binding**
*Incubation And Setup: 30 minutes*

↓

**Bead Washing**
*Low Stringency: 15 minutes*
*High Stringency: 3 x 10 minutes*

↓

**PCR Amplification**
*Incubation And Setup: 1 hour*
*Ampure: 45 minutes*
*BioAnalyzer: 1 hour; Qubit: 30 min*

**qPCR Quantification**
*Incubation & Setup: 2 hours*

↓

**Wash MiSeq**
*Running Wash 2 hours*

↓

**Pool & Denature Libraries**
*Setup: 1 hour*

↓

**Run MiSeq**
*Setup: 30 minutes*
*Run Time: 36 hours*

FIG. 1

**Fragmented DNA**

**End Repair & dA-Tail**
*Tag & T4 Polymerase + T4PNK*

**End Repaired
dA-Tailed DNA**

$p$ —————————————— $A$
$A$ —————————————— $p$

**Ligate Adapters**
*DNA Ligase + Adapters*

$p$
$T$

**Ligated DNA
(Library)**

**PCR Amplify**
*Polymerase + Primers*

**Amplified
Library**

FIG. 2

**Hybrid Selection**
*Hybridize Library to RNA Capture Baits*

**RNA Pulldown**
*Pulldown RNA Baits w/Streptavidin Beads*

**PCR Capture Library**
*Add Beads Directly To PCR Mix*

FIG. 3

• Uneven coverage
• Low off-target rate

↑ Stringency

• Even coverage
• High off-target rate

↓ Stringency

60% GC
Ex 1

30% GC
Ex 2

b  b  b

b  b

FIG. 4

Situation 1
(non-specific
interaction)

Library w/insert from
intergenic region

b

FIG. 5

FIG. 6

FIG. 7

# GENE PANEL OPTIMIZATION

## VIA IN SILICO, EMPIRICAL DESIGN

Exons with COSMIC Mutations

Filter by Most Mutations per Exon

TCGA: Filter By Patient Mutations Per Exon

2 Patients | 25 Patients

Select Based on Patient Coverage — VS

Most Broad Coverage With Fewest Exons Reduces Cost

TCGA: The Cancer Genome Atlas (1519 Patients NGS Data)

COSMIC: Catalog of Somatic Mutations Cancer (25000 Patients all genomic data)

○ Covered    ● Missed

FIG. 8

# ENABLES GREATER COVERAGE

## MINIMIZING Target Loss

**AMPLICON**

gDNA — Exon

Cancer ctDNA

PCR Enrich via Primers (Misses ctDNA Fragments / Introduces Error)

Sequencing READS

Only Amplified Reads Mutation Missed

**SMSEQ**

Exon

Capture Probe Method (Binds All Fragments >50bp)

All Unique Reads Captured Mutation Identified

FIG. 9

Europäisches Patentamt

European Patent Office

Office européen des brevets

## EUROPEAN SEARCH REPORT

Application Number

EP 19 16 8606

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2017/096394 A1 (COLOR GENOMICS INC [US]) 8 June 2017 (2017-06-08) * paragraph [0089] - paragraph [0098]; claims 1-50; examples 1-3 * | 1-15 | INV. C12Q1/6832 |
| Y | WO 2011/066388 A1 (GENISPHERE LLC [US]; GETTS ROBERT C [US] ET AL.) 3 June 2011 (2011-06-03) * paragraph [0046]; examples 3-4 * | 1-15 | |
| Y | US 2001/010910 A1 (HYLDIG-NIELSEN JENS J [US] ET AL) 2 August 2001 (2001-08-02) * examples 11,13 * | 1-15 | |
| Y | US 2007/178478 A1 (DHALLAN RAVINDER S [US] ET AL) 2 August 2007 (2007-08-02) * paragraph [1344] - paragraph [1346] * | 1-15 | |
| Y | WOOD W I ET AL: "BASE COMPOSITION-INDEPENDENT HYBRIDIZATION IN TETRAMETHYLAMMONIUM CHLORIDE: A METHOD FOR OLIGONUCLEOTIDE SCREENING OF HIGHLY COMPLEX GENE LIBRARIES", PROCEEDINGS OF THE NATIONAL ACADEMY OF SCIENCES OF THE UNITED STATES OF AMERICA, NATIONAL ACADEMY OF SCIENCES, US, vol. 82, no. 3, 1 March 1985 (1985-03-01), pages 1585-1588, XP002027508, ISSN: 0027-8424, DOI: 10.1073/PNAS.82.6.1585 * the whole document * | 1-15 | TECHNICAL FIELDS SEARCHED (IPC) C12Q |

-/--

The present search report has been drawn up for all claims

| Place of search | Date of completion of the search | Examiner |
|---|---|---|
| Munich | 5 June 2019 | Bradbrook, Derek |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 19 16 8606

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | FUCHS J ET AL: "Effects of formamide on the thermal stability of DNA duplexes on biochips", ANALYTICAL BIOCHEMISTRY, ELSEVIER, AMSTERDAM, NL, vol. 397, no. 1, 1 February 2010 (2010-02-01), pages 132-134, XP026808072, ISSN: 0003-2697 [retrieved on 2009-09-29] * the whole document * ----- | 1-15 | |

TECHNICAL FIELDS
SEARCHED      (IPC)

The present search report has been drawn up for all claims

| Place of search | Date of completion of the search | Examiner |
|---|---|---|
| Munich | 5 June 2019 | Bradbrook, Derek |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 19 16 8606

This annex lists the patent family members relating to the patent documents cited in the above-mentioned European search report.
The members are as contained in the European Patent Office EDP file on
The European Patent Office is in no way liable for these particulars which are merely given for the purpose of information.

05-06-2019

| Patent document cited in search report | | Publication date | Patent family member(s) | | Publication date |
|---|---|---|---|---|---|
| WO 2017096394 | A1 | 08-06-2017 | US 2017159040 A1<br>WO 2017096394 A1 | | 08-06-2017<br>08-06-2017 |
| WO 2011066388 | A1 | 03-06-2011 | CN 102630228 A<br>EP 2504348 A1<br>JP 2013511986 A<br>US 2010190167 A1<br>WO 2011066388 A1 | | 08-08-2012<br>03-10-2012<br>11-04-2013<br>29-07-2010<br>03-06-2011 |
| US 2001010910 | A1 | 02-08-2001 | US 6656687 B1<br>US 2001010910 A1 | | 02-12-2003<br>02-08-2001 |
| US 2007178478 | A1 | 02-08-2007 | US 2007178478 A1<br>WO 2007075836 A2 | | 02-08-2007<br>05-07-2007 |

EPO FORM P0459

For more details about this annex : see Official Journal of the European Patent Office, No. 12/82

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **XIE et al.** *Mol. Genet. Genomic Med,* 2016, vol. 4 (3), 262-272
- **BLUMENSTIEL et al.** Curr. Protoc. Hum. Genet. 2010
- **VESTHEIM et al.** *Methods Mol. Biol.,* 2011, vol. 687, 265-274
- **BENTON ; DAVIS.** *Science,* 1977, vol. 196, 180
- **GRUNSTEIN ; HOGNESS.** *Proc. Natl. Acad. Sci. U.S.A.,* 1975, vol. 72, 3961
- **AUSUBEL et al.** Current Protocols in Molecular Biology. Wiley Interscience, 2001
- **BERGER ; KIMMEL.** Guide to Molecular Cloning Techniques. Academic Press, 1987
- **SAMBROOK et al.** Molecular Cloning: A Laboratory Manual. Cold Spring Harbor Laboratory Press
- **KNIERIM et al.** *PLoS One,* 2011, vol. 6 (11), e28240
- **YANG ; HANG.** *J. Biomol. Tech.,* 2013, vol. 24 (2), 98-103
- **SAPOJNIKOVA et al.** *J. Biotechnology,* 2017, vol. 256, 1-5
- **TSOKTOURIDIS et al.** *BioTechniques,* 2005, vol. 38 (6), 885-888
- **AIGRAIN et al.** *BMC Genomics,* 2016, vol. 17 (1), 1
- **RAINE et al.** *Nucleic Acids Res.,* 2017, vol. 45 (6), e36
- **BUSTIN et al.** *Clin. Chem.,* 2009, vol. 55, 611-622
- **KARLEN et al.** *BMC Bioinformatics,* 2007, vol. 8, 131
- **RUIJTER et al.** *Methods,* 2013, vol. 59, 32-46
- **QUAIL et al.** *BMC Genomics,* 2012, vol. 13, 341
- **GNIRKE, ANDREAS et al.** Solution hybrid selection with ultra-long oligonucleotides for massively parallel targeted sequencing. *Nature Biotechnology 27.2,* 2009, 182-189
- **COUCH, FERGUS J. ; BARBARA L. WEBER.** Mutations and Polymorphisms in the familial early☐ onset breast cancer (BRCA1) gene. *Human mutation,* 1996, vol. 8.1, 8-18

- **CHONG, HANSOOK KIM et al.** The validation and clinical implementation of BRCAplus: a comprehensive high-risk breast cancer diagnostic assay. *PLoS One,* 2014, vol. 9.5, e97408
- **LINCOLN, STEPHEN E. et al.** A Systematic comparison of traditional and multigene panel testing for hereditary breast and ovarian cancer genes in more than 1000 patients. *The Journal of Molecular Diagnostics,* 2015, vol. 17.5, 533-544
- **DESMOND, ANDREA et al.** Clinical actionability of multigene panel testing for hereditary breast and ovarian cancer risk assessment. *JAMA oncology,* 2015, vol. 1.7, 943-951
- **GNIRKE, ANDREAS et al.** Solution hybrid selection with ultra-long oligonucleotides for massively parallel targeted sequencing. *Nature biotechnology,* 2009, vol. 27.2, 182-189
- **ZOOK, JUSTIN M. et al.** Extensive sequencing of seven human genomes to characterize benchmark reference materials. *bioRxiv,* 2015, 026468
- **EBERLE, MICHAEL A. et al.** A reference dataset of 5.4 million human variants validated by genetic inheritance from sequencing a three-generation 17-member pedigree. *bioRxiv,* 2016, 055541
- **ZOOK, JUSTIN M. et al.** *Integrating human sequence data sets provides a resource of benchmark SNP and indel genotype calls,* 2014
- **BRONNER, IRAAD F. et al.** Improved protocols for illumina sequencing. *Current Protocols in Human Genetics,* 2014, 18-2
- *Using a PhiX Control for HiSeq Sequencing Runs,* 11 June 2016
- **GARGIS, AMY S. et al.** Assuring the quality of next-generation sequencing in clinical laboratory practice. *Nature biotechnology,* 2012, vol. 30.11, 1033-1036
- **REHM, HEIDI L. et al.** ACMG clinical laboratory standards for next-generation sequencing. *Genetics in Medicine,* 2013, vol. 15.9, 733-747
- **RICHARDS, SUE et al.** Standards and guidelines for the interpretation of sequence variants: a joint consensus recommendation of the American College of Medical Genetics and Genomics and the Association for Molecular Pathology. *Genetics in Medicine,* 2